# EUROPEAN PATENT APPLICATION

(11) **EP 4 154 909 A1**
(43) Date of publication of application: **29.03.2023**
(21) Application number: 21795799.2
(22) Date of filing: 28.04.2021
(51) Int. Cl.: A61K 39/395, C12N 15/113, A61P 35/00, G01N 33/574

(54) **DIAGNOSIS AND TREATMENT OF CANCERS SHOWING HIGH EXPRESSION OF PIWI AND/OR NMD COMPLEX PROTEIN**

(30) Priority: 29.04.2020 CN 202010359757
(71) Applicant: ShanghaiTech University, Shanghai 201210 (CN)
(72) Inventor: LIN, Haifan, Shanghai 201210 (CN); SHI, Shuo, Shanghai 201210 (CN); YANG, Zhenzhen, Shanghai 201210 (CN)
(74) Representative: Dehns
(86) International application number: PCT/CN2021/090591
(87) International publication number: WO 2021/219009

(57) **Abstract**

The present invention provides an application of a drug or a pharmaceutical composition in the treatment of cancers showing high expression of PIWI and/or an NMD complex protein, such as gastric cancer. When applied in the treatment of cancers showing high expression of PIWI and/or an NMD complex protein, such as gastric cancer, the drug or the pharmaceutical composition of the present invention not only can effectively treat cancers, particularly gastric cancer, but also has higher specificity and will not cause harm to normal cells and body functions.

## Description

The present application claims priority to Chinese Patent Application No. 2020103597575 filed on April 29, 2020, the contents of which are incorporated herein by reference in their entireties.

### Field of technology

The invention relates to PIWI, especially PIWIL1 and/or NMD complex proteins, in the diagnosis and treatment of cancers with high expression of PIWI and/or NMD complex proteins, especially gastric cancer.

### Background of technology

Gastric cancer is one of the most common malignant tumors with a relatively poor prognosis, which is a serious threat to human health. According to statistics from the International Agency for Research on Cancer (IARC), there were about 951,000 new cases of gastric cancer worldwide in 2012, and about 723,000 deaths due to gastric cancer, ranking fifth in the incidence rate of malignant tumors and third in the mortality rate. In 2012, China had about 424,000 new cases of gastric cancer and 298,000 deaths, ranking second in the incidence of malignant tumors(1). Multidisciplinary comprehensive treatment based on surgery and chemotherapy is the main treatment method for gastric cancer. Total gastrectomy or subtotal gastrectomy combined with D2 lymphadenectomy has been gradually accepted as the standard surgical method for gastric cancer. For patients with advanced gastric cancer who cannot undergo radical surgery, chemotherapy is the main means to prolong their survival (2). Although the overall survival rate is improved after neoadjuvant chemotherapy, the overall response rate is low and the prognosis of patients is poor. Therefore, gene targeted therapy, as a new method for the treatment of gastric cancer, especially advanced gastric cancer, has attracted much attention in recent years. There are currently only three FDA-approved drugs for targeted therapy of gastric cancer: Trastuzumab, Ramucirumab and Pembrolizumab. Trastuzumab targets HER2 (3, 4), Ramucirumab targets VEGFR2(5-7), Pembrolizumab targets PD-1(8). Although these drugs have made some breakthroughs in the targeted therapy of other cancer types, their effect is not ideal in the treatment of gastric cancer, especially advanced gastric cancer, and there are still great limitations in clinical application. Moreover, the genes that these drugs target are widely expressed in normal cells and bodies, and play important physiological functions, which will inevitably cause damage to normal cell and body functions when they are targeted. Therefore, it is urgent to find and identify a target that is more specifically expressed in gastric cancer cells, but not expressed or rarely expressed in normal gastric cells or tissues, so that the treatment of gastric cancer with this gene as the target can not only effectively kill gastric cancer cells, but also minimize the damage to normal tissues and cells.

PIWI (P-element-induced Wimpy testis) protein is a branch of Argonaut superfamily. The PIWI protein family consists of four members of PIWIL1 (PIWI like RNA-mediated gene silencing 1), PIWIL2, PIWIL3 and PIWIL4 in human, three members of PIWIL1, PIWIL2 and PIWIL4 in mouse, and PIWI in fruit flies. PIWI was first discovered by Professor Haifan Lin in fruit flies in 1997. It's named for its famous mutant phenotype p-element-induced wimpy testis inactivation (P-element-induced wimpy testis) induced by transposons (9). PIWI plays an important role in the maintenance and self-renewal of germline stem cells. PIWI proteins bind to a class of small non-coding RNAs called PIWI-interacting RNAs (piRNA) (10-13). The known classical regulation of PIWI is the combination of PIWI and piRNA to form a effecting factor complex, i.e., piRNA-induced silencing complex, this complex is involved in epigenetic regulation, post-transcriptional regulation and other molecular regulation, and is essential for germline development, gametogenesis, stem cell self-renewal, transposon suppression and genome integrity (14-19). PIWI protein is expressed in a variety of organisms from sponge to human. In these organisms, PIWI protein and piRNA are mainly expressed in the germline. Human PIWI protein is mainly expressed in the male germline and basically not expressed in the somatic tissues outside the germline. It has been reported that PIWI, such as PIWIL1, is highly expressed in various tumor tissues, including gastric cancer tissues (20-23). However, the prior art has not disclosed PIWI has a function to promote tumorigenesis and metastasis, especially gastric tumor, both *in vivo* and *in vitro,* and it is not clear whether the regulation mechanism of PIWI in cancer depends on piRNA to play its biological function. Therefore, it is not possible to define the application of PIWI in targeted cancer therapy.

### Contents of the invention

The technical problem to be solved in the present invention is to overcome the defects in the prior art such as the lack of effective medicaments for the treatment of cancers (e.g. gastric cancer) with high expression of PIWI and/or NMD complex proteins, the present invention provides a use of a medicament and a pharmaceutical composition in the treatment of cancers (e.g. gastric cancer) with high expression of PIWI and/or NMD complex proteins. When the medicament or pharmaceutical composition of the present invention is used to treat cancer (such as gastric cancer) with high expression of PIWI and/or NMD complex proteins, it can not only effectively treat cancer, especially gastric cancer, but also has higher specificity, and will not cause harm to normal cell and body functions.

In the prior art, only PIWI, such as PIWIL1, has been reported to be highly expressed in various tumor tissues, including gastric cancer tissues. However, researchers in this field are not clear that PIWI, such as PIWIL1, has the function of promoting tumor formation and metastasis *in vivo* in cancers with high expression of PIWI, especially gastric cancer. Moreover, the classical regulation mode of PIWI known from the prior art is that PIWI binds with piRNA to form effector complex, namely piRNA-induced silencing complex. To achieve the purpose of inhibiting PIWI, researchers in this field usually consider preparing inhibitors targeting both PIWI and piRNA. However, piRNA belongs to the category of small RNAs with a wide variety and short sequence, so it is very difficult to design inhibitors that targets piRNA. However, the inventor unexpectedly found in lots of experiments that there was almost no piRNA in cancer cells with high expression of PIWI, especially gastric cancer cells. PIWI, such as PIWIL1, can regulate the growth and migration of cancer cells with high expression of PIWI, especially gastric cancer cells, in a manner that is not dependent on piRNA, but coordinated with NMD complex, which breaks traditional viewpoints or technical prejudices in this field. Therefore, by designing medicaments that target PIWI and/or NMD complex, medicaments that block the binding of PIWI and NMD complex proteins, medicaments that reduce the expression of PIWI and/or medicaments that reduce the expression of NMD complex proteins, the purpose of effectively treating cancers with high expression of PIWI, especially gastric cancer, can be achieved. Besides, there is no need to design difficult inhibitors that target piRNA.

In order to solve the technical problems described above, the first aspect of the present invention provides a medicament that blocks the binding of PIWI and NMD complex proteins for one or more of the following purposes:
(a)to diagnose and/or treat cancers with high expression of PIWI and/or NMD complex proteins;
(b)to inhibit growth of cancer cells;
(c)to inhibit progression of cancer cell cycle;
(d)to inhibit migration of cancer cells;
(e)to inhibit tumorigenicity of cancer cells;
(f)to inhibit metastasis of cancer cells *in vivo;*
(g)to regulate cell cycle signaling pathways of cancer cells;
(h)to regulate focal adhesion and adherens junction signaling pathways of cancer cells.

Preferably, the cancer is gastric cancer, such as early or advanced gastric cancer.

Preferably, PIWI comprises PIWIL1, PIWIL2, PIWIL3 and/or PIWIL4.

Preferably, the NMD complex proteins comprise UPF1, UPF2, SMG1, UPF3, SMG5, SMG6 and/or SMG7, and preferably UPF1, UPF2 and/or SMG1.

Preferably, the medicament is a medicament that blocks the binding of PIWI and NMD complex proteins by mutating and/or inhibiting the binding site of PIWI and NMD complex proteins. Preferably, the medicament is a medicament that blocks the binding of PIWI and NMD complex proteins by mutating and/or inhibiting the binding site of the PIWI that binds to the NMD complex proteins. Preferably, the medicament is a medicament that blocks the binding of PIWI and NMD complex proteins by mutating and/or inhibiting the binding site of the NMD complex that binds to the PIWI.

Preferably, the medicament is an inhibitor designed for the binding site of the PIWI to the NMD complex proteins. Preferably, the medicament is an inhibitor designed for the binding site of PIWI to the NMD complex proteins such as a PIWIL1 inhibitor, a PIWIL2 inhibitor, a PIWIL3 inhibitor and/or a PIWIL4 inhibitor. Preferably, the medicament is an inhibitor designed for the binding site of the NMD complex proteins to PIWI such as UPF1 inhibitor, UPF2 inhibitor, SMG1 inhibitor, UPF3 inhibitor, SMG5 inhibitor, SMG6 inhibitor and/or SMG7 inhibitor, preferably UPF1, UPF2 and/or SMG1 inhibitor.

Preferably, the medicament is a small molecule (may be a small molecule inhibitor, generally a competitive small molecule inhibitor), for example, a small molecule chemical substance such as a small molecule compound, or a small molecule biological substance such as a small molecule active peptide. Wherein, the small-molecule compound preferably exists in a form of a pharmaceutically acceptable salt of the compound, a solvent complex of the compound, a solvent complex of a pharmaceutically acceptable salt of the compound, or a crystalline form of the compound.

Preferably, the medicament is a macromolecular substance, for example, a macromolecular biological substance such as a polysaccharide, a protein such as an antibody, a nucleic acid, or a macromolecular chemical substance such as a polymer compound.

Preferably, the medicament is an extract of traditional Chinese medicine and/or animals and plants.

In order to solve the technical problems described above, the second aspect of the present invention provides a medicament that targets PIWI and/or NMD complex proteins for one or more of following purposes:
(a)to diagnose and/or treat cancers with high expression of PIWI and/or NMD complex proteins;
(b)to inhibit the growth of cancer cells;
(c)to inhibit the progression of cancer cell cycle;
(d)to inhibit the migration of cancer cells;
(e)to inhibit tumorigenicity of cancer cells;
(f)to inhibit metastasis of cancer cells *in vivo;*
(g)to regulate cell cycle signaling pathways of cancer cells;
(h)to regulate focal adhesion and adherens junction signaling pathways of cancer cells.

Preferably, PIWI comprises PIWIL1, PIWIL2, PIWIL3 and/or PIWIL4.

Preferably, the NMD complex proteins comprise UPF1, UPF2, SMG1, UPF3, SMG5, SMG6 and/or SMG7, and preferably UPF1, UPF2 and/or SMG1.

Preferably, the medicament that targets PIWI are PIWI inhibitors, such as PIWIL1 inhibitors, PIWIL2 inhibitors, PIWIL3 inhibitors, and/or PIWIL4 inhibitors.

Preferably, the medicament that targets NMD complex proteins are inhibitors of NMD complex proteins, such as UPF1 inhibitors, UPF2 inhibitors, SMG1 inhibitors, UPF3 inhibitors, SMG5 inhibitors, SMG6 inhibitors, and/or SMG7 inhibitors, such as NMDI-14, NMDI1 and/or VG1.

Preferably, the cancer is gastric cancer, such as early or advanced gastric cancer.

Preferably, the medicament is a small molecule (may be a small molecule inhibitor, generally a competitive small molecule inhibitor), for example, a small molecule chemical substance such as a small molecule compound, or a small molecule biological substance such as a small molecule active peptide. Wherein, the small-molecule compound preferably exists in a form of a pharmaceutically acceptable salt of the compound, a solvent complex of the compound, a solvent complex of a pharmaceutically acceptable salt of the compound, or a crystalline form of the compound.

Preferably, the medicament is a macromolecular substance, for example, a macromolecular biological substance such as a polysaccharide, a protein such as an antibody, a nucleic acid, or a macromolecular chemical substance such as a polymer compound.

Preferably, the medicament is an extract of traditional Chinese medicine and/or animals or plants.

In order to solve the technical problems described above, the third aspect of the present invention provides a medicament that reduces the expression of PIWI and/or NMD complex proteins for one or more of the following purposes:
(a) to diagnose and/or treat cancers with high expression of PIWI and/or NMD complex proteins;
(b)to inhibit growth of cancer cells;
(c)to inhibit progression of cancer cell cycle;
(d)to inhibit migration of cancer cells;
(e)to inhibit tumorigenicity of cancer cells;
(f)to inhibit metastasis of cancer cells *in vivo;*
(g)to regulate cell cycle signaling pathways of cancer cells;
(h)to regulate focal adhesion and adherens junction signaling pathways of cancer cells.

Preferably, the medicament that reduces PIWI expression is a medicament that silences, downregulates, and/or knocks out PIWI gene.

Preferably, the medicament that reduces the expression of NMD complex proteins is medicament that silences, downregulates, and/or knocks out genes of NMD complex proteins.

Preferably, the PIWI comprises PIWIL1, PIWIL2, PIWIL3 and/or PIWIL4.

Preferably, the NMD complex proteins comprise UPF1, UPF2, SMG1, UPF3, SMG5, SMG6 and/or SMG7, and preferably UPF1, UPF2 and/or SMG1.

Preferably, the cancer is gastric cancer, such as early or advanced gastric cancer.

Preferably, the medicament is a small molecule (may be a small molecule inhibitor, generally a competitive small molecule inhibitor), for example, a small molecule chemical substance such as a small molecule compound, or a small molecule biological substance such as a small molecule active peptide. Wherein, the small-molecule compound preferably exists in a form of a pharmaceutically acceptable salt of the compound, a solvent complex of the compound, a solvent complex of a pharmaceutically acceptable salt of the compound, or a crystalline form of the compound.

Preferably, the medicament is a macromolecular substance, for example, a macromolecular biological substance such as a polysaccharide, a protein such as an antibody, a nucleic acid, or a macromolecular chemical substance such as a polymer compound.

Preferably, the medicament is an extract of traditional Chinese medicine and/or animals or plants.

The medicament mentioned above in the first aspect, second aspect and/or third aspect may be a medicament that does not comprise piRNA targeting, such as piRNA inhibitors; and/or, the pharmaceutical composition does not comprise a medicament that blocks the binding of PIWI to piRNA.

Preferably:
The medicament that blocks binding of PIWI and piRNA is a medicament that blocks binding of PIWI and piRNA by mutating and/or inhibiting binding site of PIWI and piRNA, preferably a medicament that blocks the binding by mutating and/or inhibiting the binding site of the PIWI that binds to the piRNA and/or a medicament that blocks the binding by mutating and/or inhibiting the binding site of the piRNA that binds to the PIWI.

Preferably:
The medicament that blocks the binding of PIWI and piRNA is an inhibitor designed for the binding site of PIWI and piRNA, preferably an inhibitor designed for the binding site of PIWI that binds to the piRNA, and/or an inhibitor designed for the binding site of piRNA that binds to the PIWI.

In order to solve the technical problems described above, the fourth aspect of the present invention provides a pharmaceutical composition comprising the medicament as described in the first aspect, second aspect and/or third aspect of the present invention.

Preferably, the pharmaceutical composition is used for one or more of the following purposes:
(a)to diagnose and/or treat cancers with high expression of PIWI and/or NMD complex proteins;
(b)to inhibit growth of cancer cells;
(c)to inhibit progression of cancer cell cycle;
(d)to inhibit migration of cancer cells;
(e)to inhibit tumorigenicity of cancer cells;
(f)to inhibit metastasis of cancer cells *in vivo;*
(g)to regulate cell cycle pathways of cancer cells;
(h)to regulate focal adhesion and adherens junction signaling pathways of cancer cells.

Preferably, the PIWI comprises PIWIL1, PIWIL2, PIWIL3 and/or PIWIL4.

Preferably, the NMD complex proteins comprise UPF1, UPF2, SMG1, UPF3, SMG5, SMG6 and/or SMG7, and preferably UPF1, UPF2 and/or SMG1.

Preferably, the cancer is gastric cancer, such as early or advanced gastric cancer.

Preferably, the pharmaceutical composition does not comprise medicaments that target piRNA, such as piRNA inhibitors.

Preferably, the pharmaceutical composition does not comprise medicament that blocks the binding of PIWI to piRNA.

Wherein, the medicament that blocks the binding of PIWI and piRNA can be a medicament that blocks the binding of PIWI and piRNA by mutating and/or inhibiting the binding site of PIWI and piRNA, preferably a medicament that blocks the binding by mutating and/or inhibiting the binding site of the PIWI that binds to the piRNA and/or a medicament that blocks the binding by mutating and/or inhibiting the binding site of the piRNA that binds to the PIWI.

Wherein, the medicament blocking the binding of PIWI and piRNA may be an inhibitor designed for the binding site of PIWI and piRNA, preferably an inhibitor designed for the binding site of PIWI that binds to the piRNA, and/or an inhibitor designed for the binding site of piRNA that binds to the PIWI.

Preferably, the pharmaceutical composition further comprises pharmaceutical adjuvant.

Preferably, the pharmaceutical composition further comprises other medicaments; the other medicaments are medicaments for the diagnosis and/or treatment of cancers with high expression of PIWI and/or NMD complex proteins.

Preferably, the pharmaceutical composition consists of one or more of the medicaments as described in the first aspect, second aspect and/or third aspect of the present invention.

In order to solve the technical problems described above, the fifth aspect of the present invention provides a method for diagnosing and/or treating cancers with high expression of PIWI and/or NMD complex proteins, the method comprises diagnosis and/or treatment using the medicament as described in the first aspect, second aspect and/or third aspect of the present invention and/or the pharmaceutical composition as described in the fourth aspect of the present invention.

Preferably, the cancer is gastric cancer, such as early or advanced gastric cancer.

Preferably, the PIWI comprises PIWIL1, PIWIL2, PIWIL3 and/or PIWIL4.

Preferably, wherein the medicament and/or pharmaceutical composition is used for diagnosis and/or treatment by one or more of the following mechanisms:
(a)to inhibit growth of cancer cells;
(b)to inhibit progression of cancer cell cycle;
(c)to inhibit migration of cancer cells;
(d)to inhibit tumorigenicity of cancer cells;
(e)to inhibit metastasis of cancer cells *in vivo;*
(f)to regulate cell cycle pathways of cancer cells;
(g)to regulate focal adhesion and adherens junction signaling pathways of cancer cells.

In order to solve the technical problems described above, the sixth aspect of the present invention provides a use of the medicament as described in the first aspect, second aspect and/or third aspect and/or the pharmaceutical composition as described in the fourth aspects of the present invention in the diagnosis and/or treatment of cancers with high expression of PIWI and/or NMD complex proteins.

Preferably, the cancer is gastric cancer, such as early or advanced gastric cancer.

Preferably, the PIWI comprises PIWIL1, PIWIL2, PIWIL3 and/or PIWIL4.

Preferably, the medicament and/or pharmaceutical composition is used for diagnosis and/or treatment by one or more of the following mechanisms:
(a)to inhibit growth of cancer cells;
(b)to inhibit progression of cancer cell cycle;
(c)to inhibit migration of cancer cells;
(d)to inhibit tumorigenicity of cancer cells;
(e)to inhibit metastasis of cancer cells *in vivo;*
(f)to regulate cell cycle signaling pathways of cancer cells;
(g)to regulate focal adhesion and adherens junction signaling pathways of cancer cells.

In order to solve the technical problems described above, the seventh aspect of the present invention provides the medicament as described in the first aspect, second aspect and/or third aspect and/or the pharmaceutical composition as described in the fourth aspects of the present invention in the preparation of a drug for the diagnosis and/or treatment of gastric cancer. Specifically: a use of the medicament that blocks the binding of PIWI and NMD complex proteins (as described in the first aspect) and/or the pharmaceutical composition comprising the medicament (as described in the fourth aspect) in the preparation of a drug for the diagnosis and/or treatment of gastric cancer is provided; a use of the medicament that targets PIWI and/or NMD complex proteins (as described in the second aspect), and/or a pharmaceutical composition comprising the medicament (as described in the fourth aspect) in the preparation of a drug for the diagnosis and/or treatment of gastric cancer is provided. The medicament that reduces the expression of PIWI and/or NMD complex proteins (as described in the third aspect), and/or the pharmaceutical composition comprising the medicament (as described in the fourth aspect) in the preparation of a drug for the diagnosis and/or treatment of gastric cancer.

Preferably, the gastric cancer is early or advanced gastric cancer.

Preferably, the PIWI comprises PIWIL1, PIWIL2, PIWIL3 and/or PIWIL4.

Preferably, the medicament and/or pharmaceutical composition is used for diagnosis and/or treatment by one or more of the following mechanisms:
(a)to inhibit growth of cancer cells;
(b)to inhibit progression of cancer cell cycle;
(c)to inhibit migration of cancer cells;
(d)to inhibit tumorigenicity of cancer cells;
(e)to inhibit metastasis of cancer cells *in vivo;*
(f)to regulate cell cycle signaling pathways and focal adhesion and adherens junction signaling pathways of cancer cells.

In order to solve the technical problems described above, the eighth aspect of the present invention provides a method for inhibiting growth of cancer cells, inhibiting operation of cancer cell cycle, inhibiting migration of cancer cells, inhibiting tumorigenesis ability of cancer, inhibiting metastasis ability of cancer, regulating signaling pathways of cancer cell cycle and/or focal adhesion and adherens junction signaling pathways of cancer cells, wherein the method comprises inhibition and/or regulation using the medicament as described in the first aspect, second aspect and/or third aspect of the present invention and/or the pharmaceutical composition as described in the fourth aspect of the present invention.

Preferably, the cancer is gastric cancer, such as early or advanced gastric cancer.

Preferably, the PIWI comprises PIWIL1, PIWIL2, PIWIL3 and/or PIWIL4.

In order to solve the technical problems described above, the ninth aspect of the present invention provides a use of the medicament as described in the first aspect, second aspect and/or third aspect and/or the pharmaceutical composition as described in the fourth aspect of the present invention in inhibiting growth of cancer cells, inhibiting operation of cancer cell cycle, inhibiting migration of cancer cells, inhibiting tumorigenesis ability of cancer, inhibiting metastasis ability of cancer, regulating signaling pathways of cancer cell cycle and/or focal adhesion and adherens junction signaling pathways of cancer cells.

Preferably, the cancer is gastric cancer, such as early or advanced gastric cancer.

Preferably, the PIWI comprises PIWIL1, PIWIL2, PIWIL3 and/or PIWIL4.

In order to solve the technical problems described above, the tenth aspect of the present invention provides a use of the medicament as described in the first aspect, second aspect and/or third aspect of the present invention and/or the pharmaceutical composition as described in the fourth aspect of the present invention in preparing a drug for inhibiting growth of cancer cells, inhibiting operation of cancer cell cycle, inhibiting migration of cancer cells, inhibiting tumorigenesis ability of cancer, inhibiting metastasis ability of cancer, regulating signaling pathways of cancer cell cycle and/or focal adhesion and adherens junction signaling pathways of cancer cells.

Preferably, the cancer is gastric cancer, such as early or advanced gastric cancer.

Preferably, the PIWI comprises PIWIL1, PIWIL2, PIWIL3 and/or PIWIL4.

In order to solve the technical problems described above, the eleventh aspect of the present invention provides a use of PIWI and/or NMD complex proteins in screening medicaments as described in the first aspect, second aspect and/or third aspect of the present invention and/or the pharmaceutical composition as described in the fourth aspect of the present invention.

Preferably, the PIWI comprises PIWIL1, PIWIL2, PIWIL3 and/or PIWIL4.

Preferably, the NMD complex proteins comprise UPF1, UPF2, SMG1, UPF3, SMG5, SMG6 and/or SMG7, and preferably UPF1, UPF2 and/or SMG1.

In order to solve the technical problems described above, the twelfth aspect of the present invention provides a use of cell lines highly expressing PIWI and/or NMD complex proteins in screening medicaments s described in the first aspect, second aspect and/or third aspect of the present invention and/or the pharmaceutical composition as described in the fourth aspect of the present invention.

Preferably, the medicament is a small molecule (may be a small molecule inhibitor, generally a competitive small molecule inhibitor), for example, a small molecule chemical substance such as a small molecule compound, or a small molecule biological substance such as a small molecule active peptide; the small-molecule compound preferably exists in a form of a pharmacologically acceptable salt of the compound, a solvent complex of the compound, a solvent complex of a pharmacologically acceptable salt of the compound, or a crystalline form of the compound.

Preferably, the medicament is a macromolecular substance, for example, a macromolecular biological substance such as a polysaccharide, a protein such as an antibody, a nucleic acid, or a macromolecular chemical substance such as a polymer compound.

Preferably, the medicament is an extract of traditional Chinese medicine and/or of animals or plants.

Preferably, the cell lines are gastric cancer cell lines, preferably SNU-1, SNU-16 and/or AGS. For the first time, the applicant found that SNU-1, SNU-16 and other cell lines were the cell lines with high expression of PIWII,1.

Preferably, the PIWI comprises PIWIL1, PIWIL2, PIWIL3 and/or PIWIL4.

Preferably, the NMD complex proteins comprise UPF1, UPF2, SMG1, UPF3, SMG5, SMG6 and/or SMG7, and preferably UPF1, UPF2 and/or SMG1.

In order to solve the technical problems described above, the thirteenth aspect of the present invention provides a use of PIWI and/or NMD complex proteins as biomarkers and/or therapeutic targets for cancers with high expression of PIWI and/or NMD complex proteins.

Preferably, the PIWI comprises PIWIL1, PIWIL2, PIWIL3 and/or PIWIL4.

Preferably, the NMD complex proteins comprise UPF1, UPF2, SMG1, UPF3, SMG5, SMG6 and/or SMG7, and preferably UPF1, UPF2 and/or SMG1.

Preferably, the cancer is gastric cancer, such as early or advanced gastric cancer.

In order to solve the technical problems described above, the fourteenth aspect of the present invention provides a biomarker for detection and/or diagnosis of cancers with high expression of PIWI and/or NMD complex proteins, wherein the biomarker comprises PIWI and/or NMD complex proteins.

Preferably, the PIWI comprises PIWIL1, PIWIL2, PIWIL3 and/or PIWIL4.

Preferably, the NMD complex proteins comprise UPF1, UPF2, SMG1, UPF3, SMG5, SMG6 and/or SMG7, and preferably UPF1, UPF2 and/or SMG1.

Preferably, the cancer is gastric cancer, such as early or advanced gastric cancer.

In order to solve the technical problems described above, the fifteenth aspect of the present invention provides a kit for the detection and/or diagnosis of cancers with high expression of PIWI and/or NMD complex proteins, wherein the kit comprises antibodies for detection of PIWI and/or NMD complex proteins.

Preferably, the PIWI comprises PIWIL1, PIWIL2, PIWIL3 and/or PIWIL4.

Preferably, the NMD complex proteins comprise UPF1, UPF2, SMG1, UPF3, SMG5, SMG6 and/or SMG7, and preferably UPF1, UPF2 and/or SMG1.

Preferably, the cancer is gastric cancer, such as early or advanced gastric cancer.

In the present invention, nonsense-mediated mRNA decay (NMD) is an essential eukaryotic process that regulates transcript quality and abundance and is associated with a variety of processes, including embryonic development and cancer progression. The NMD complex protein has three core components: UPF1(Regulator of nonsense transcripts 1), UPF2(Regulator of nonsense transcripts 2) and UPF3(Regulator of nonsense transcripts 3). Phosphorylation of UPF1(core NMD component) by SMG1(Serine/threonine-protein kinase) is essential for NMD.

In the present invention, the medicament that targets NMD complex proteins can be inhibitors of NMD complex protein, for example NMDI-14(Ethyl 2-(((6,7-dimethyl-3-oxo-1,2,3,4-tetrahydro-2-quinoxalinyl)acetyl)amino)-4,5-dimethyl-3-thiophenecarboxylate, Nonsense-Mediated mRNA Decay Inhibitor 14), NMDI14 targets the pocket of SMG7 protein, and disrupts the SMG7-UPF1 interaction; for example NMDI1 (inhibits the binding of UPF1 to SMG5) and/or for example VG1 (inhibits the binding of UPF1 to SMG5)(Victoria J. B. Gotham, etc., Synthesis and activity of a novel inhibitor of nonsense-mediated mRNA decay, Organic & Biomolecular Chemistry, 2016, 14, 1559-1563).

In the present invention, the PIWI protein family has four members of PIWIL1 (piwi like RNA-mediated gene silencing 1 [*Homo sapiens* (human)], https://www.ncbi.nlm.nih.gov/gene/9271, gene ID: 9271), PIWIL2, PIWIL3 and PIWIL4, in mouse there are only PIWIL1, PIWIL2 and PIWIL4, and in Drosophila there is only PIWI (see https://www.ncbi.nlm.nih.gov/gene/?term=PIWI).

In the present invention, the "medicament that targets a substance (such as protein)" can be understood as a medicament that can bind with a substance (such as protein). "Inhibitor that targets a substance (e.g., PIWI proteins or NMD complex proteins)" can be understood as a preparation that has a function of inhibition upon binding to a substance (e.g., PIWI proteins or NMD complex proteins).

In the present invention, "include, comprise or contain" may mean that there are other components in addition to those listed next; it can also mean "be composed of...", which means that only the following listed components are included and no other components exist.

On the basis of conforming to the common knowledge in this field, the above optimal conditions can be arbitrarily combined to obtain the better embodiments of the present invention.

The reagents and raw materials used in the present invention are commercially available.

Positive progress effect of this invention: When the medicament and pharmaceutical composition of the present invention are used to treat cancers with high expression of PIWI and/or NMD complex proteins, especially gastric cancer, they can not only effectively treat cancers with high expression of PIWI and/or NMD complex proteins, but also have higher specificity, and will not cause harm to normal cell and body functions.

### Description of Drawings

Figure 1 shows mRNA expression of PIWIL1 in six different human gastric cancer cell lines and one normal gastric epithelial cell line.
Figure 2 shows protein expression of PIWIL1 in six different human gastric cancer cell lines, one normal gastric epithelial cell line and positive control of mouse testis.
Figure 3 shows the immunofluorescence results, which indicates that PIWIL1 protein was mainly localized in the cytoplasm of gastric cancer cell.
Figure 4 shows the relative PIWIL1 mRNA levels in gastric cancer samples from 97 patients as compared to their paired normal tissues.
Figure 5 shows the protein expression of PIWIL1 and the location of PIWIL1 protein in 104 paired samples of gastric cancer tissue microarray.
Figure 6 shows that PIWIL1 expression is positively correlated with tumor grade and metastasis, and negatively correlated with the degree of tumor differentiation.
Figure 7 shows the successful knockout of PIWIL1 in gastric cancer cell SNU-1 using CRISPR-Cas9 technology.
Figure 8 shows that knockout of PIWII,1 at either normal serum concentration or low serum concentration (starvation) significantly inhibited the growth of gastric cancer cell SNU-1, and the inhibition effect was more obvious at the low serum concentration of 5%FBS.
Figure 9 shows that knockout of PIWII,1 inhibited cell cycle G1/S transition in gastric cancer cells.
Figure 10 shows that knockout of PIWIL1 inhibited cell migration of gastric cancer cells
Figure 11 shows that knockout of PIWIL1 inhibits subcutaneous tumorigenesis of gastric cancer cells in nude mice.
Figure 12 shows that knockout of PIWII,1 inhibited metastasis of gastric cancer cell in nude mice.
Figure 13 shows that RNA-Seq data from WT and PIWIL1-KO SNU-1 cells analyzed by weighted gene co-expression network analysis (WGCNA) identified 41 co-expressing modules, and the genes of 41 co-expressing modules were found to be regulated by PIWIL1.
Figure 14 shows that among all the gene modules regulated by PIWII,1, the blue module and turquoise module are the two modules of genes most related to PIWIL1 trait and most significantly regulated by PIWIL1. After analyzing the genes of these two modules , it was found that the cell cycle pathway, focal adhesion and adherens junction signaling pathways of cancer cells were most significantly enriched in these two modules.
Figure 15 shows that the mRNA expression of some important oncogenic genes related to cell cycle is inhibited by PIWIL1 knockout; the mRNA expression of some important tumor suppressor genes related to cell migration is promoted by PIWIL1 knockout.
Figure 16 shows that in gastric cancer cell SNU-1 with high expression of PIWI L1, piRNA expression could hardly be detected, and even piRNA enriched by PIWIL1 could not be detected.
Figure 17 shows that PIWIL1 mutant lacking piRNA binding ability can still regulate PIWIL1 downstream target genes and play an oncogenic function as wild-type PIWIL1 in gastric cancer.
Figure 18 shows that experimental results of PIWIL1 immunoprecipitation coupled Mass-Spectrum (PIWIL1-IP MS) shows that PIWIL1 could bind to UPF1 protein.
Figure 19 shows that PIWIL1 immunoprecipitated nonsense mRNA decay (NMD) complex proteins UPF1, UPF2, SMG1 and activated UPF1 (phosphorylated UPF1).
Figure 20 shows immunofluorescence results, which indicates that PIWIL1 could colocalize with UPF1 in P-bodies which the NMD pathway works in.
Figure 21 shows the specific binding region of PIWII,1 responsible for binding to UPF1, and that disruption of this binding region significantly disrupted the binding of PIWIL1 to UPF1, whereas disruption of piRNA binding site did not affect the binding of PIWIL1 to UPF1.
Figure 22 shows that loss of binding ability to UPF1 significantly affected the expression regulation of some downstream tumor suppressor genes related to cell migration by PIWIL1, and significantly affected the regulation of cell migration by PIWIL1.

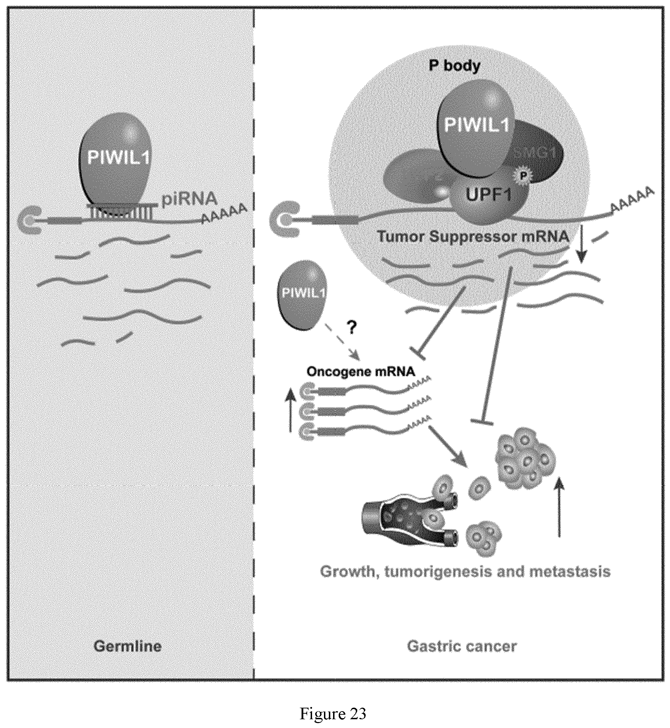

Figure 23 shows a pattern diagram, which exhibits that different from the co-function of PIWI-piRNA complex formed by PIWI protein and piRNA in the germline, piRNA, the classic partner of PIWIL1, is almost absent in gastric cancer cells. PIWIL1 regulates the growth, migration, tumorigenesis and metastasis of gastric cancer cells through other piRNA-independent pathways, such as NMD pathway in gastric cancer.

### Specific implementation mode

The invention is further described below by means of Examples without thereby limiting the present invention to the scope thereof. The experimental methods not specified in the following embodiments shall be selected according to the conventional methods and conditions, or according to the product instructions.

### Example 1 PIWIL1 was highly expressed in gastric cancer cells and tissues

### 1.1 mRNA expression of PIWIL1 in gastric cancer cells

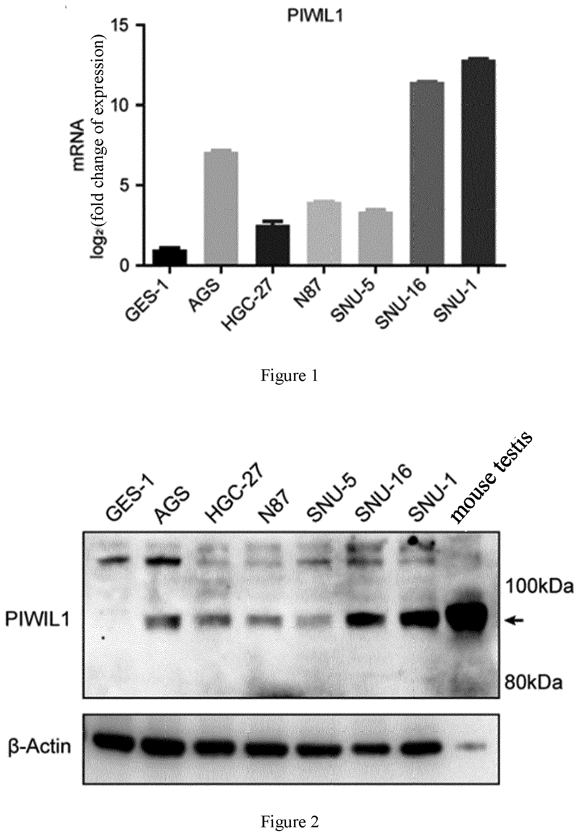

RNA was extracted from six different gastric cancer cell lines (AGS, HGC-27, N87, SNU-5, SNU-16 and SNU-1) and a normal gastric epithelial cell line (GES-1). The corresponding cDNA was obtained by reverse transcription, and the mRNA expression of PIWIL1 and β-Actin was detected by quantitative RT-PCR. The relative expression level of PIWIL1 in each sample was calculated using the expression level of β-Actin as the internal standard to normalize the level of PIWIL1 from the same sample (Ct_{PIWIL1} - Ct_{Actin} = Δ*Ct*_{PIWIL1}). The mRNA level of GES-1 was normalized to 1, the ratio of the relative expression of PIWIL1 in each gastric cancer cell ( ΔCt_{PIWIL1_{T}}) to the relative expression of PIWIL1 of GES-1 ( ΔCt_{PIWIL1*_{N}*}) was calculated, that is, the expression multiple of PIWIL1 in each gastric cancer cell higher or lower than that of GES-1 (ΔCt_{PIWIL1_{T}} - ΔCt_{PIWIL1*_{N}*} = ΔΔ*Ct*_{PIWIL1}). Based on the relative multiplication of these seven cell lines, the bar graph of Figure 1 was made. Ct values of each quantitative RT-PCR sample were averaged from biological replicates of three independent samples. Figure 1 shows that the mRNA expression of PIWIL1 in different gastric cancer cells is higher than that in normal gastric epithelial cells.

### 1.2 Expression and localization of PIWII,1 protein in gastric cancer cells

Proteins were extracted from six different gastric cancer cell lines (AGS, HGC-27, N87, SNU-5, SNU-16 and SNU-1) and a normal gastric epithelial cell line (GES-1). The protein expressions of PIWIL1 and β-Actin were detected by Western Blot. Figure 2 shows that the protein expression of PIWIL1 in different gastric cancer cells is higher than that in normal gastric epithelial cells.

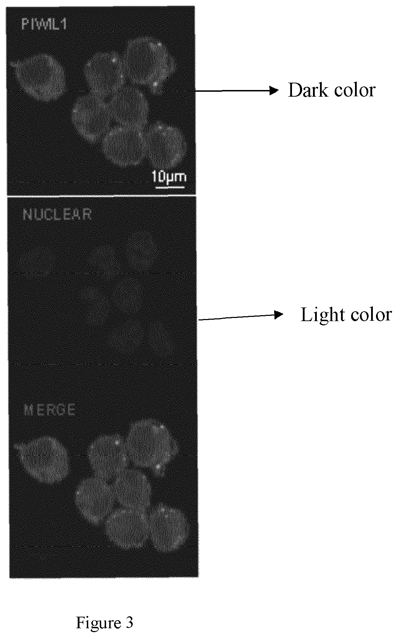

Immunofluorescence assay was performed in the cell line SNU-1 with the highest mRNA and protein expression of PIWIL1, and PIWIL1 antibody was used as the primary antibody. In Figure 3, the dark fluorescence signal is the protein signal of PIWII,1, and the light fluorescence signal is the nuclear DAPI signal, Figure 3 shows that PIWIL1 is largely expressed in the cytoplasm of gastric cancer cells.

### 1.3 mRNA expression of PIWII,1 in gastric cancer tissues

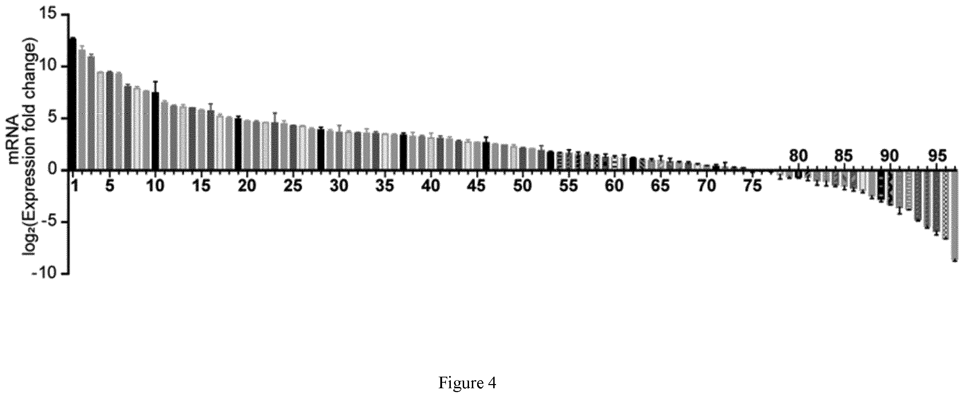

97 pairs of clinical samples of gastric tumor tissues and normal tissues were collected, RNA was extracted and the corresponding cDNA was obtained by reverse transcription. The mRNA expression levels of PIWIL1 and β-Actin were detected by quantitative RT-PCR. The expression level of β-Actin was used as the internal standard in each sample, the relative expression of PIWIL1 in each sample was calculated (Ct_{PIWIL1} - Ct_{Actin} = Δ*Ct*_{PIWIL1}), The relative expression of PIWIL1 in tumor tissue samples of each pair of samples ΔCt_{PIWIL1_{T}} was compared with the relative expression of PIWIL1 in normal tissue samples ΔCt_{PIWIL1*_{N}*} to obtain the differential expression of PIWIL1 in each pair of samples. Based on the differential expression fold of PIWIL1 in each pair of samples, the column of Figure 1 was made. A value of Y coordinate >1 in Figure 2 indicates that the mRNA expression of PIWIL1 in tumor tissue samples in each pair of samples is more than two times higher than that in the corresponding normal tissue samples. Ct values of each quantitative RT-PCR sample were averaged using three technical replicates. Figure 4 shows that in 63 out of 97 paired gastric cancer samples, PIWIL1 mRNA expression was twice in the tumor than that in the normal tissue.

### 1.4 Expression and localization of PIWIL1 protein in gastric cancer

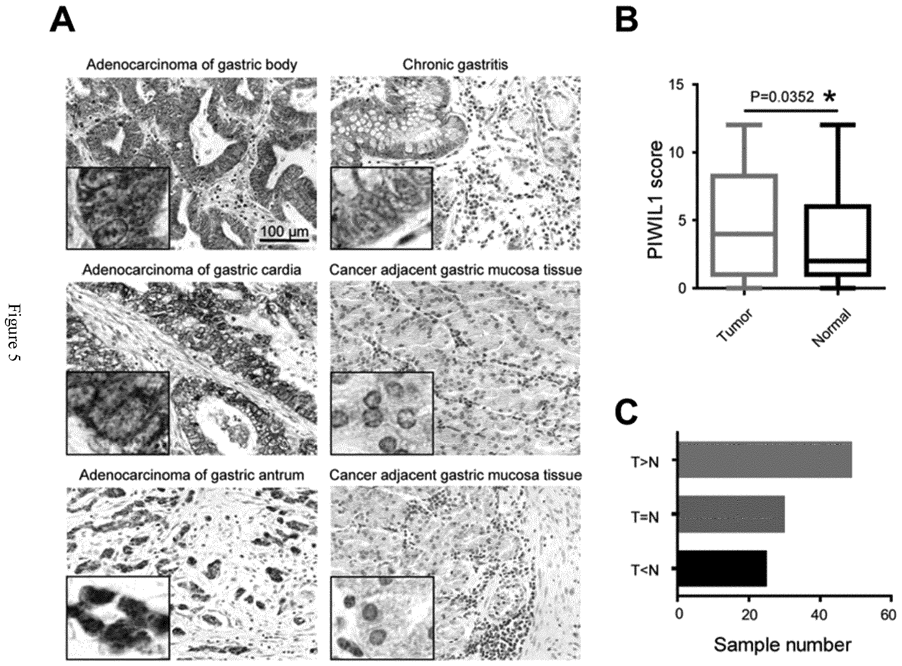

The expression of PIWIL1 protein in tissue microarrays representing 104 gastric cancer patients was examined by immunohistochemistry (IHC) staining. The brown signal was the positive signal of PIWIL1. The left panel (A) of Figure 5 is a representative IHC of PIWIL1 compared with paired normal tissues, which shows that PIWIL1 protein expression in gastric cancer tissues is significantly higher than that in paired normal tissues, and most of the PIWIL1 signal is in the cytoplasm. The box plot (B) in the upper right panel of Figure 5 shows IHC scores tissue microarrays of PIWII,1 expression in 104 pairs of paired tumor and normal tissues in the gastric cancer. Each tissue spot on the tissue microarray was scored by stain strength (range from 0 to 3) and the percentage of PIWIL1-positive cells (range from 0 to 4), respectively. By scoring the positive scores of PIWIL1 immunohistochemical staining, it was found that PIWIL1 score in gastric cancer tissues was significantly higher than that in the paired normal tissues. The bar graph (C) in the lower right panel of Figure 5 shows by counting the number of tissue pairs in which the IHC score is higher than, equal to, and lower than that in the paired normal tissue, respectively, the sample number in the T>N group (the IHC score is higher than that in the paired normal tissue) is significantly larger than the sample number in the two other groups.

### 1.5 The expression of PIWIL1 in gastric cancer is closely related to the clinicopathological progression of gastric cancer

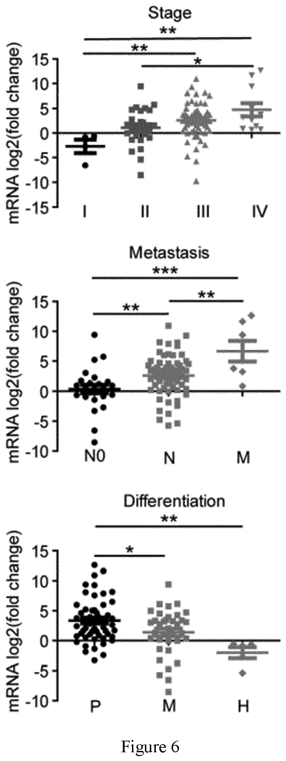

The mRNA expression of PIWIL1 in 97 paired clinical patient samples was correlated and analyzed with pathological information (tumor stage, TNM metastasis, and differentiation degree) of these patient samples, Figure 5 shows the expression of PIWIL1 mRNA in gastric cancer samples is significantly positive correlated with both metastasis and the tumor-node-metastasis (TNM) stage, the higher expression of the PIWIL1 mRNA, the lower the differentiation of gastric cancer and the more malignant the tumor. While the expression of PIWIL1 mRNA negative correlated with the degree of differentiation. These results suggest that the expression of PIWIL1 is closely related to the malignancy and tumor progression of gastric cancer. In Figure 6, I, II, III, and IV denote stages 1-4. N0: no nodes metastasis are involved; N: lymph node metastasis including N1, N2, and N3; M: distant metastasis. In the lower graph: P: poor differentiation; M: moderate differentiation; H: high differentiation. SE represents standard error.

In conclusion, it can be seen from this Example that PIWILI is specifically highly expressed in clinical tissues of gastric cancer patients and various gastric cancer cell lines compared with normal tissues and cell lines. The present invention proves for the first time that the gastric cancer cell line SNU-1 is a cell line with very high expression of PIWIL1. The gastric cancer cell line SNU-1 with high abundance of PIWIL1 expression can be an effective tool for screening new medicament that targets PIWIL1. The selected molecules may be (but are not limited to) proteins, antibodies, small chemical molecules, extracts of traditional Chinese medicine, and extracts of animal or plant. In addition, this Example shows that the higher the expression of PIWIL1, the higher the pathological stage of gastric cancer patients; the higher the expression of PIWIL1, the higher the degree of metastasis; the higher the expression of PIWIL1, the lower the degree of differentiation, which shows that the expression of PIWILI is closely related to the clinical outcome of gastric cancer, indicating that PIWIL1 is expected to be a biomolecular marker for the diagnosis and classification of gastric cancer.

### Materials and Methods

### RNA extraction and quantitative RT-PCR

Total RNA was isolated by TRIzol (Invitrogen) for the RNA extraction methods of the samples in steps 1.1 and 1.2, according to the instructions of the manufacturer. For reverse transcription, we used the ABI High Capacity cDNA Reverse Transcription kit (Life Technologies, 4368814). Quantitative RT-PCR reactions were performed according to the instructions of the Bio-Rad real-time PCR system (iQTM SYBR Green Supermix and CFX96TM real-time system). Quantitative PCR primers are listed in Table 1.

### Cell culture and clinical samples

SNU-5 cells were cultured in IMDM medium (ThermoFisher Scientific, 31980030) supplemented with 20% fetal bovine serum. SNU-1, SNU-16, N87, and HGC-27 cells were cultured in RPMI 1640 medium (ThermoFisher Scientific, 61870036) supplemented with 10%fetal bovine serum. AGS cells were cultured in ATCC-formulated F-12K Medium (ATCC, 30-2004) supplemented with 10% fetal bovine serum. GES-1 cells were cultured in DMEM medium (ThermoFisher Scientific, 11995065) supplemented with 10% fetal bovine medium. All these cell lines were incubated at 37 °C with 5% CO₂.

97 pairs of clinical samples were purchased from the tissue bank of the Institute of Health Sciences, Chinese Academy of Sciences. The local ethics committee approved the study, and the regulations of this committee were followed.

### Western blotting analysis

Total proteins were extracted by lysis buffer [20mM Tris-HCl pH7.4, 150mMNaCl, 1 % (v/v) IGEPAL^{®} CA-630 (Millipore SIGMA, Cat# 18896), 1mM EDTA, 0.5 mM DTT, cOmplete^{™} EDTA-free Protease Inhibitor Cocktail Tablets (MERCK, Cat# 4693132001), and PhosStop Tablets (MERCK, Cat# 4906837001)]. Protein samples were diluted (3:1) with a 4 × Laemmli sample buffer (Biorad, Cat# 1610747) and heated at 98 °C for 5 min. Proteins were isolated by the TGX Fast Cast acrylamide kit, 7.5% or 10% (Bio-Rad, 1610173TA) at 120 V, and electrotransferred to a PVDF membrane (Merck/Millipore, IPVH00010) at 0.3A for 1.5 h. The membrane was blocked with 5% DifcoTM skim milk (BD Biosciences, 232100) at room temperature for 2 h, which was diluted with TBS (Bio-Rad, 1706435) supplemented with 0.1% Tween 20 (Santa Cruz Biotechnology, sc-29113). PIWIL1 antibody (Abcam, ab181056) was used at 1:1000 dilutions.

### Immunofluorescence microscopy

2×10⁵ cells were seeded on a coverslip (Fisher, 12-545-83) in a 24-well plate. After 24 h, cells were washed three times in PBS and then fixed in 4% formaldehyde (paraformaldehyde powder, 95%, 158127-2.5KG, Sigma) at room temperature for 15 min. The fixed cells were washed in ice-cold PBST (1% Tween 20 in PBS) three times (5 min each wash), blocked in 3% BSA in PBST at room temperature for 2 h, and washed in PBST once, and then incubated with anti-PIWII,1 (Abcam, Cat# ab181056, 1:100 dilution), anti-hDcp1a (56-Y) antibodies (Santa Cruz Biotechnology, sc-100706, 1:500 dilution), and anti-UPF1 (Cell Signaling TECHNOLOGY, Cat# 12040, 1:200 dilution) in 3% BSA at 4 °C overnight. After incubation, cells were washed five times in PBST, 5 min each time. Cells were incubated with the secondary antibody of FITC-conjugated AffiniPure goat anti-mouse IgG (H+L) (Jackson ImmunoResearch Laboratories, Cat# 115-095-003, 1:100 dilution) or FITC-conjugated AffiniPure goat anti-rabbit IgG (H+L) (Jackson ImmunoResearch Laboratories, Cat# 111-095-003, 1: 100 dilution) or Alexa Fluor 594-conjugated AffiniPure goat anti-mouse IgG (H+L) (Jackson ImmunoResearch Laboratories, 115-585-003, Cat# 1:400 dilution) or Alexa Fluor 680 (ThermoFisher Scientific, Cat# A10043, 1:500 dilution) highly cross-absorbed with donkey anti-rabbit IgG (H+L) diluted in PBST containing 3 % BSA at room temperature for 2 h, followed washing once with PBST for 5 min. DAPI (Life Technologies, D1306, 1:5000 dilution) was then added to the PBST buffer and incubated at room temperature for 10 min, followed by washing three times in PBST, 5 min each time. Coverslips were removed one at a time, and we added one drop of FluorPreserveTM (Merck/Millipore, Cat# 345787-25MLCN), mounted them to the glass slide, pressed gently, sealed them with nail polish, and stored them at 4°C overnight before confocal immunofluorescence microscopy (Zeiss, LSM710).

### Immunohistochemistry

Human gastric cancer tissue chip (Cat# HStm-Can090PT-01) was purchased from Shanghai Superchips Company. Mouse tumors for immunohistochemistry were harvested from xenograft mice, cut into 10-micrometer-thick consecutive sections, and mounted on glass slides. After deparaffinized, rehydrating, antigen retrieval, and blocking endogenous peroxidases, the sections or tissue chip were washed three times in PBS for 5 mins each and blocked for 1 h in 0.01 mol/L PBS supplemented with 0.3% Triton X-100 and 5% normal goat serum, followed by addition of anti-PIWIL1 antibody (Atlas antibody, HPA018798, 1:1000 dilution), or anti-PCNA antibody (Servicebio, GB11010-1, 1:1000 dilution), or anti-Ki67 antibody (Servicebio, GB13030-2, 1:300 dilution) at 4°C overnight. After brief washing in 0.01 mol/L PBS, sections were exposed for 2 hours to 0.01 mol/L PBS containing horseradish peroxidase-conjugated rabbit anti-goat immunoglobulin G (1:500), followed by development with 0.003% H₂O₂ and 0.03% 3,3'-diaminobenzidine in Tris-HCl (pH 7.6). Immunohistochemistry for each sample was performed at least three separate times, and all sections were counterstained with hematoxylin.

### Example 2 PIWIL1 promotes gastric cancer cell growth, migration, tumorigenesis and metastasis in vivo

### 2.1 PIWIL1 was successfully knocked out in gastric cancer cell SNU-1 by CRISPR-Cas9 technology

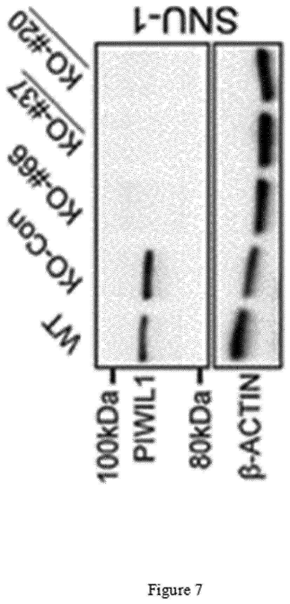

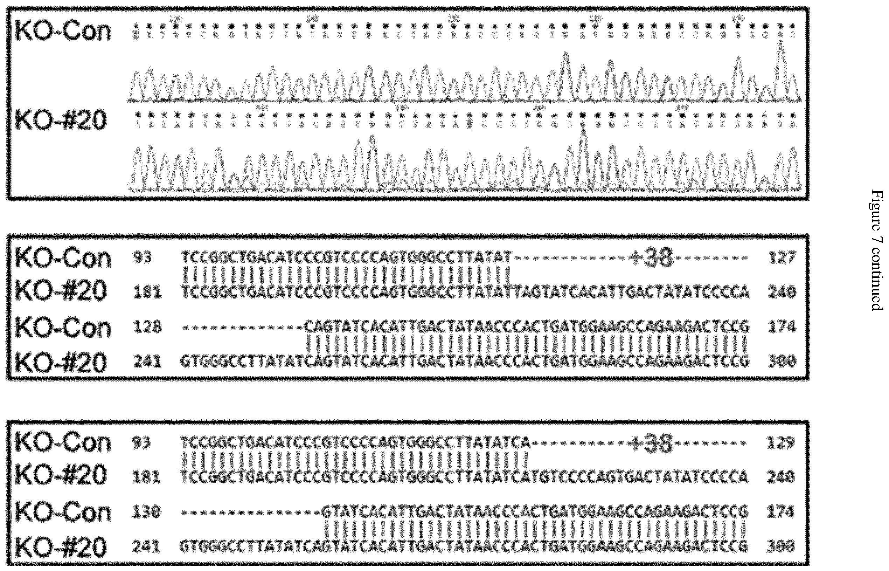

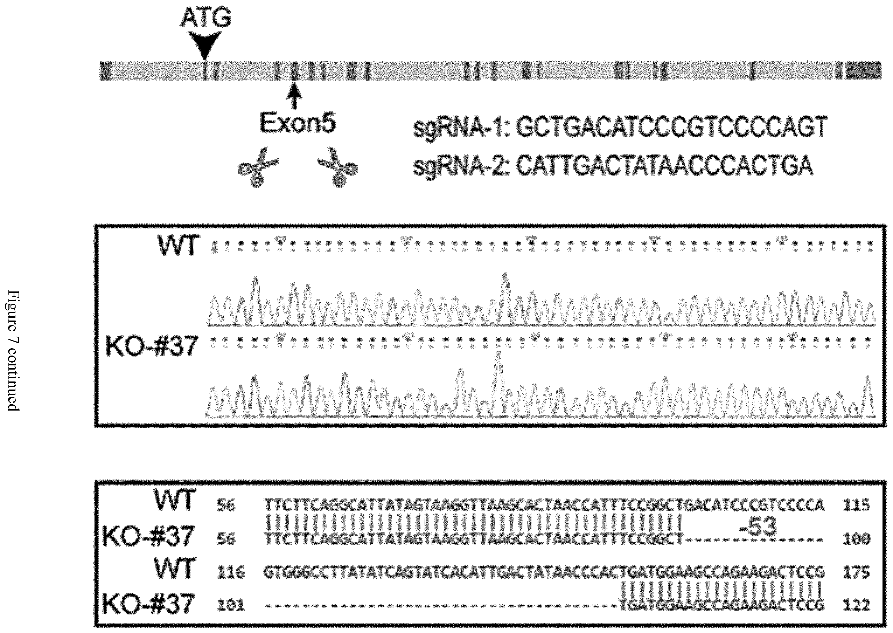

Apair of sgRNAs were cloned into a pGL3-U6-sgRNA-PGK-puromycin vector. pGL3-U6-sgRNA-PGK-puromycin and pST1374-N-NLS-flag-linker-Cas9-D10A were gifts from Professor Xingxu Huang (Addgene plasmid # 51133; http://n2t.net/addgene:51133; RRID: Addgene: 51133). Based on the method (24) of Professor Huang Xingxu's Nature Methods article, the constructed PIWII,1-sgRNAplasmid and Cas9 plasmid were co-transferred into SNU-1 cells. 48 hours after transfection, Puromycin and Blasticidin S were added for cell clone selection. The genomes of the selected cell clones were extracted, and genomic PCR was performed for the identification of PIWIL1-knockout cell clone. The PCR products were sequenced for the first generation, and the sequence of the wild-type PIWIL1 gene was compared to determine whether there were any deletions or insertional mutations in the PIWIL1 gene sequence of cell clones that resulted in frameshift of the amino acid encoding PIWIL1 protein, leading to the premature stop code of PIWIL1 translation. By Western blotting assay and DNA sequencing of PIWIL1 mutant cell clones, we found that we successfully knocked out PIWIL1 in gastric cancer cell line SNU-1(Figure 7). The sgRNA and PCR primers for knocking out PIWIL1- genomes are listed in Table 2.

### 2.2 Knockout of PIWII,1 inhibited the growth of gastric cancer cells

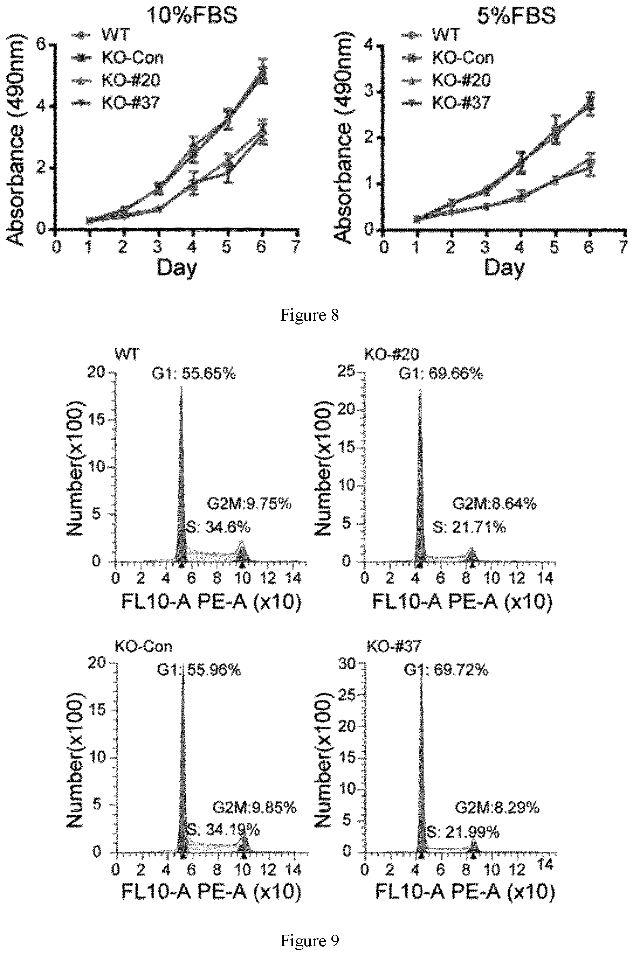

We analyzed the growth curve of SNU-1 cell clones with or without PIWIL1-KO in normal 10%FBS or starvation 5%FBS cell culture medium conditions by MTS assay in biological triplicates (SNU-1 cell clone without PIWIL1-knockout: Wide type (WT) and Knockout control (KO-Con); SNU-1 cell clone with PIWIL1-knockout: PIWIL1-knockout clone #20 and PIWIL1-knockout clone #37). The growth of cells with and without PIWIL1 knockout were compared. Figure 8 shows that PIWIL1 knockdown significantly inhibited the growth of gastric cancer cells, especially under the condition of serum starvation.

### 2.3 Knockout of PIWIL1 inhibited the cell cycle of gastric cancer cells

Cell cycle PI-staining assay of PIWIL1-WT (WT and KO-Con) or PIWIL1-KO (KO-#20 and KO-#37) SNU-1 cells were performed by Flow Cytometry. Figure 9 shows that knockout of PIWIL1 significantly inhibited the G1/S phase transition of gastric cancer cells SNU-1.

### 2.4 Knockout of PIWIL1 inhibited cell migration of gastric cancer cells

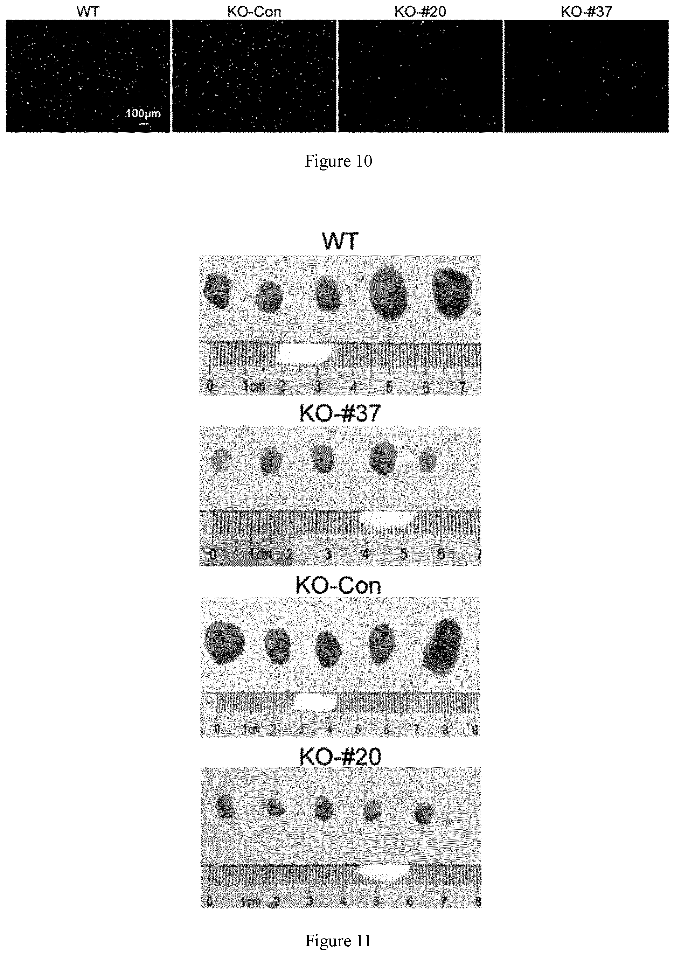

By Transwell migration assay, cells of PIWIL1 wild-type (WT), PIWIL1 knockout control (KO-Con), PIWIL1 knockout cell clone #20 and PIWIL1 knockout cell clone #37 were photographed and observed, and the number of migrating cells with green fluorescent dye was counted. Figure 10 shows that knockout of PIWIL1 significantly inhibited the cell migration of gastric cancer cell SNU-1.

### 2.5 Knockout of PIWIL1 inhibited tumorigenesis of gastric cancer cells in vivo

PIWIL1 wild-type (WT), PIWIL1 knockout control (KO-Con), PIWIL1 knockout cell clone #20 and PIWIL1 knockout cell clone #37 were subcutaneously injected into nude mice, five mice in each group. Body weight and tumor growth were observed once a week. Figure 11 shows that the tumorigenesis ability of PIWIL1-knockout gastric cancer cells in mice is much lower than that of wild-type PIWIL1 gastric cancer cells.

### 2.6 Knockout of PIWIL1 inhibited the metastasis of gastric cancer cells in vivo PIWIL1

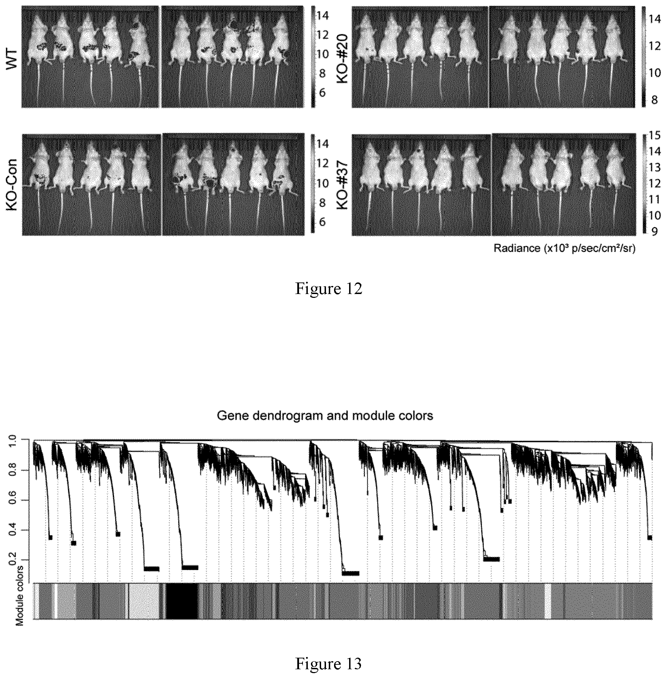

PIWIL1 wild-type (WT), PIWIL1 knockout control (KO-Con), PIWIL1 knockout cell clone #20 and PIWIL1 knockout cell clone #37 were infected with virus with both luciferase and GFP (green fluorescence). Luciferase and GFP double positive cells were obtained by flow cytometry. The four groups of positively labeled cells were injected into nude mice through the tail vein, five mice in each group. The fluorescence intensity of luciferase, which represents the size of tumor metastases in mice, was monitored by photographing every week. Figure 12 shows that the metastasis ability of PIWIL1 knockout gastric cancer cells in mice is much lower than that of wild-type PIWIL1 gastric cancer cells.

In conclusion, this Example shows that knockout of PIWIL1 in gastric cancer cells can significantly inhibit cell growth, cell cycle and cell migration, and effectively inhibit tumorigenesis and metastasis of gastric cancer cells in nude mice.

### Materials and Methods

### Cell proliferation assays

Cell proliferation was determined using CellTiter 96_AQueous MTS (3-(4,5-dimethylthiazol-2-yr)-5-(3-carboxymethoxyphenyl)-2-(4-sulfophenyl)-2H tetrazolium, inner salt) reagent powder (Promega, G1111) according to the instructions of the manufacturer. Absorbance at 490-nm wavelength was read using EnSpire multimode plate readers (PerkinElmer Life Sciences).

### Transwell migration assay

The transwell assay was performed using Corning FluoroBlokTM cell culture inserts (Falcon, 351152) according to the instructions of the manufacturer. 1×10⁵ cells were seeded in each well. After three hours, cells were stained and photographed.

### In vivo tumorigenicity assay

Five-week-old male nude mice were housed under standard conditions. SNU-1 gastric cancer cells were harvested and washed with PBS and suspended in RPMI 1640 medium without serum. 5×10⁶ of these cells were injected subcutaneously into the right flank of nude mice. Tumor growth was measured every 7 days, and tumor volume was estimated as length × width × height × 0.5236. Tumors were harvested from ether-anesthetized mice. All procedures conformed with the Guide for the Care and Use of Laboratory Animals (NIH publication No. 80-23, revised in 1996), and approved by the Animal Care and use Committee, Shanghai Tech University.

### In vivo metastasis assay

The SNU-1 gastric cancer cell line was labeled with luciferase-expressing lentivirus containing an independent open reading frame of GFP. Luciferase expression was determined by using luciferin (Xenogen, Alameda, CA) and an *in vivo* imaging system (Xenogen). The luciferase-expressing SNU-1 of PIWII,1-WT or PIWII,1-KO cells (1×10⁶ in 100 µl PBS) were injected through the tail vein, after which the overall health condition and bodyweight of the mice were monitored. The metastatic lesions were monitored every other week. An aqueous solution of luciferin (150 mg/kg intraperitoneally) was injected 10 min before imaging, and then the mice were anesthetized with Forane (Abbott). The mice were placed into a light-tight chamber of the CCD camera system (Xenogen), and the photons emitted from the luciferase-expressing cells within the animal were quantified for 1 min using the software program Living Image (Xenogen) as an overlay on Igor (Wavemetrics, Seattle, WA). All procedures conformed with the Guide for the Care and Use of Laboratory Animals (NIH publication No. 80-23, revised in 1996) and approved by the Animal Care and use Committee, Shanghai Tech University.

### Example 3 PIWIL1 regulates many tumor-related signaling pathways, especially cell cycle signaling pathway and focal adhesion and adherens junction signaling pathways signaling pathway

3.1 RNA was extracted from cells of PIWIL1 wild-type (WT), PIWIL1 knockout control (KO-Con), PIWIL1 knockout cell clone #20 and PIWIL1 knockout cell clone #37.

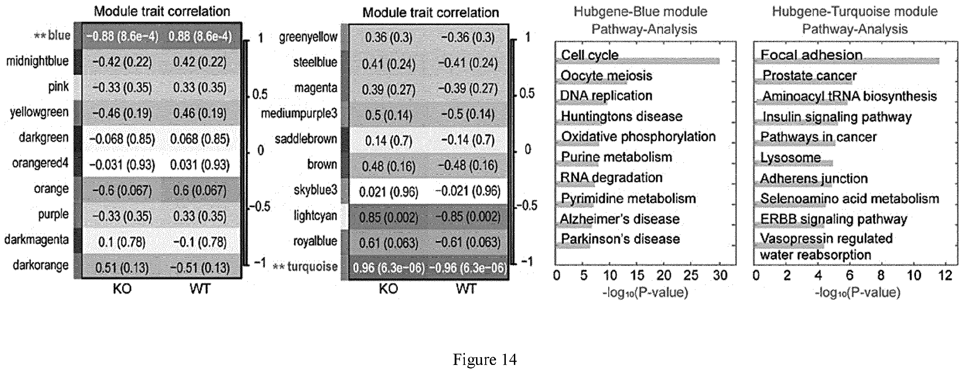

These five groups were classified into WT/KO20#, KO-Con/KO-37#, WT/37#, KO-Con/KO-20# and WT/KO20#, and RNA-Seq was performed. We further analyzed RNA-Seq data from these five groups using weighted gene co-expression network analysis (WGCNA), Figure 13 shows that 41 co-expressing modules were identified to be regulated by PIWIL1.We then investigated the correlation between PIWIL1 trait and the co-expressed gene modules, the red color in the heat map of Figure 13 represents the positive correlation between gene modules and trait, while the blue color represents the negative correlation, each cell contains the corresponding correlation value and significance *p*-value, based on this, the heat map in Figure 14 shows that two gene clusters of blue module and turquoise module are significantly regulated by PIWIL1, the blue module genes were positively regulated by PIWIL1, while the turquoise module genes were negatively regulated by PIWIL1. Hub genes with kME≥0.9 in the gene clusters of these two modules were analyzed by KEGG pathway. It was found that the hub genes of the blue module were highly enriched in cell cycle and DNA replication pathways, while the hub genes of the turquoise module were highly enriched in focal adhesion and adherens junction signaling pathways.

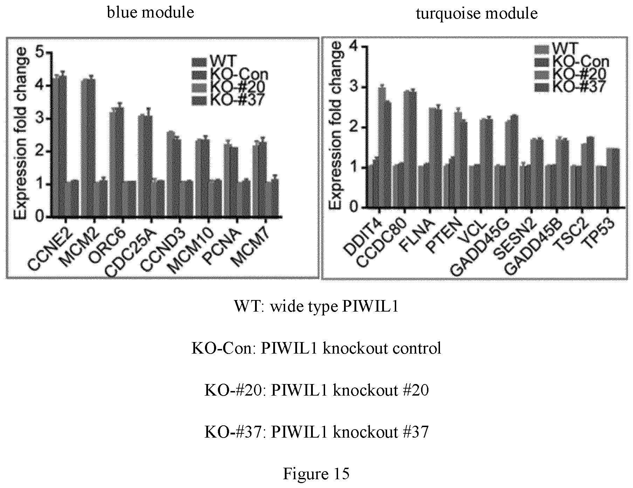

3.2 Quantitative PCR was used to quantify the mRNA expression of 8 well-documented oncogenes in the blue module and 10 well-documented tumor suppressor genes in the turquoise module, Figure 15 shows that the mRNA expression of eight cell cycle-related genes was inhibited by PIWIL1 knockout, the mRNA expression of 10 tumor suppressor genes related to cell adhesion was promoted by PIWIL1 knockout.

This embodiment shows that transcriptomic studies have found that PIWIL1 can regulate cell cycle and focal adhesion signaling pathways related to the biological phenotype of gastric cancer, and promote the expression of many oncogenes and inhibit the expression of many tumor suppressor genes related to the progression of gastric cancer.

### Materials and Methods

### Total RNA-deep sequencing without rRNA and weighted gene co-expression network analysis (WGCNA) analysis

100ng of total RNA from each cell sample was used for deep sequencing of whole transcriptome without ribosomal RNA(rRNA). The NEBNext^{®} Ultra II Directional RNA Library Prep Kit for Illumina^{®} was used to construct the library in accordance with the kit's instructions. The libraries were quality controlled with a Bioanalyzer 2200 (Agilent, Santa Clara, CA) and sequenced by HiSeq X (Illumina, San Diego, CA) on a 150 bp paired-end run, clean data were obtained from the raw reads by removing the adaptor sequences, and low-quality reads. Then, the clean data were then aligned to the human genome (GRCh38, NCBI) using Hisat2 (25) software. Htseq (26) software was used to get gene counts, and the RPKM method was used to determine the gene expression. WGCNA (27) was performed across all samples using the standard method with a power of 17 to cluster the expression patterns. WGCNA was used to construct a network in published data sets independently and generated an independent list of hub genes (kME > 0.9) for each data set. RUVseq algorithm was used for batch-to-batch correction.

### Example 4 PIWIL1 plays its oncogenic function and regulation in gastric cancer in a piRNA-independent manner

### 4.1 piRNA was not found in gastric cancer cells with high PIWIL1 expression

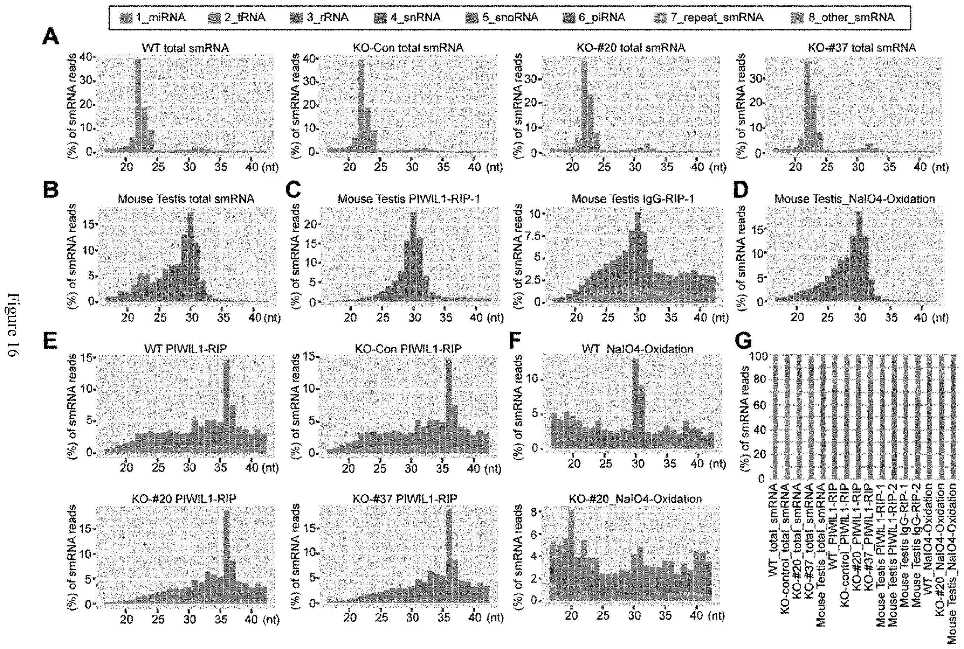

Although it has been well demonstrated that PIWI protein family carries out various biological functions by interacting with piRNAs in germline, we detected piRNA expression in our gastric cancer cell line SNU-1 with high PIWIL1 expression to determine whether PIWIL1 can have piRNA-independent regulation and biological function in gastric cancer. Deep sequencing of small RNA was performed on wild-type and PIWIL1-knockout gastric cancer cells, piRNAs were enriched by immunoprecipitation of PIWIL1 antibody. We used the methods of immunoprecipitation of PIWIL1 antibody and NaIO₄ oxidation treatment to determine whether there are any *bona fide* piRNAs that PIWIL1 binds with were detected in gastric cancer cell line with high expression of PIWIL1.Figure16 shows that although there was a large amount of piRNA expression in the mouse testis in the systemically positive control, piRNA could hardly be detected in gastric cancer cells with high expression of PIWIL1.

### 4.2 PIWIL1 protein without piRNA binding ability still plays the same oncogenic biological function and regulation as wild-type PIWIL1 protein in gastric cancer

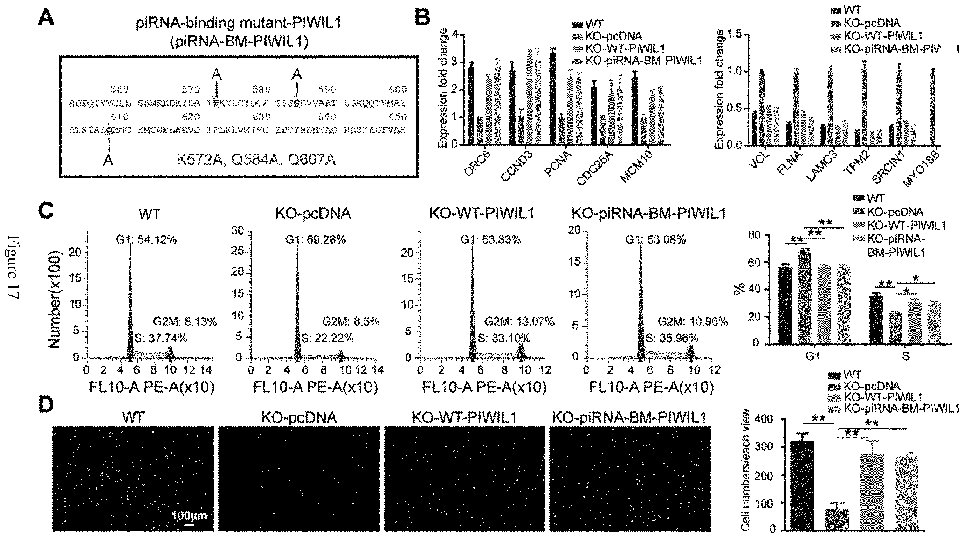

Based on previous studies, it was concluded that lysine at 572, glutamine at 572, and glutamine at 607 of human PIWIL1 are evolutionally conserved piRNA binding site (28-29). We constructed PIWIL1 expression plasmid with lack of piRNA binding ability by mutating these three amino acids into alanine. The wild-type PIWIL1 expression plasmid and the PIWIL1 expression plasmid lacking piRNA binding ability were respectively transferred into PIWIL1 knockout SNU-1 gastric cancer cell clone, and the complement experiment was conducted to investigate the effect of the loss of piRNA binding ability on the regulation of PIWIL1 on its downstream target genes, especially those related to tumor progression, and to investigate the effect of loss of piRNA binding ability on PIWIL1 on cell cycle and migration of cancer cells. Figure 17 shows that when PIWIL1 loses piRNA binding ability, it can still regulate oncogenes related to cell cycle and tumor suppressor genes related to cell-cell adhesion like wild-type PIWII,1. Furthermore, Figure 17 shows that PIWIL1 without piRNA binding ability can still rescue the cell cycle arrest and cell migration inhibition induced by PIWIL1 knockdown as well as wild-type PIWIL1.

In conclusion, this Example shows that PIWIL1 does not depend on piRNA to regulate gastric cancer cell cycle operation and cell migration. Meanwhile, PIWIL1 can also regulate the expression of many important oncogenes and tumor suppressor genes in a piRNA independent manner, especially genes related to cell cycle and cell migration phenotype. Therefore, these piRNA-independent biological functions and gene expression regulation of PIWIL1 are expected to make PIWIL1 a good target for gastric cancer therapy, especially for gastric cancer patients with rapid tumor growth and metastasis, and the effect of piRNA should not be considered for PIWIL1-targeted therapy in such patients.

### Materials and Methods

### Small RNA-Seq library construction and sequencing

Small RNA libraries were prepared from the extracted 1µg total RNA or 100ng PIWIL1-antibody-pulled down RNA using NEBNext Multiplex Small RNA Library Prep Set from Illumina11 (Set 1) (NEB, Cat# E7300S) according to the manufacturer's instructions. The libraries were sequenced using the Illumina HiSeqX platform for 150-nucleotide pair-end runs.

### Construction of Small RNA-Seq library using β-elimination by NaIO4-oxidation

20µg total RNA was oxidized to achieve β-elimination by exposing to 20 mM NaIO₄ (SigmaAldrich, Cat# 311448) in 200 mM lysine-HCl buffer (pH 8.5, Sigma-Aldrich, Cat# 62929) in a total volume of 40 µl at 37 °C for 30 min with shaking in a dark tube. The reaction was quenched by 2 µl ethylene glycol. The RNA was column-purified (RNA Clean & Concentrator-5, Zymo, Cat# R1015) and eluted in 8 µl molecular biology grade, RNase-free water. 6µl eluted RNA was used for constructing Small RNA libraries by NEBNext Multiplex Small RNA Library Prep Set (Set 1) (NEB, Cat# E7300S) according to the manufacturer's instructions. The libraries were sequenced using the Illumina HiSeqX platform for 150-nucleotide pair-end runs.

### Analyses of small RNA sequencing data

Small RNA sequences were processed with TrimGal ore (version 0.4.4_dev; http://www.bioinformatics.babraham.ac.uk/projects/trim_galore/) with the default adapter trimming mode to auto-detect adapters in the 150bp long sequencing reads. A size cutoff using 17bp to 42 bp was applied to retain small RNA reads of suitable length. The commands were: "trim_galore --phred33 --gzip -q 20 --fastqc --output_dir trimmed_fqc_out --length 17-max_length 42 --trim-n $read". Subsequently trimmed reads were mapped onto the corresponding genome [human (hg38) or mouse (mm10)] using bowtie1 (version 1.2.1.1) (30) with commands "bowtie -S -p 5 -v 0 -n 0 -l 18 -k 1 --no-unal --al $GMAPPED_FQ Homo_sapiens. GRCh38.dna.primary_assembly.fa $read > $GMAPPED_SAM". Genomemapped reads were sequentially mapped to the libraries of miRNA, tRNA, rRNA, snRNA, snoRNA, piRNAs, and repetitive elements. miRNA libraries were obtained from miRBase (March 11, 2018) (31). tRNA reference fasta was built by combining the tRNA sequences from GtRNAdb (32) and those from genome annotation. rRNA libraries were prepared from RNAcentral (January of 2019) (33). cDNA libraries of both snRNA and snoRNA were obtained from both genome annotation and RNAcentral (January of 2019). piRNAs were classified as *de novo* and known, with the former identified by proTRAC (version 2.4.2) (34), and the latter by mapping onto piRNAs from piRNABank. Commands for proTRAC were: "perl proTRAC _2.4.2.pl -genome Homo_sapiens. GRCh38.dna.primary_assembly.header.simple.fa - map $map_file -repeatmasker hg38.repeatMasker.matched.gtf -geneset Homo_sapiens.GRCm38.95.chr.gtf -pimin 25 -pimax 31". $mapfile was the mapped output using sRNAmapper (version 1.0.4) (35). Repeat-derived small RNAlibraries were obtained from the RepeatMasker annotation in UCSC genome browser.

### Wild-type PIWIL1 and the piRNA binding mutant PIWIL1 cDNA cloning

Total RNA was used for cDNA synthesis by SuperScript_III reverse transcriptase (Invitrogen, 18080044) according to the instructions of the manufacturer. The cDNA was used as a template for amplification by Phusion high-fidelity DNApolymerase (New England Biolabs, M0530L) in PCR and PIWIL1 was cloned into the pcDNA3.1-3xFlag.

The cloning of the piRNA-binding mutant PIWIL1 gene was carried out by the KOD-PlusMutagenesis kit (TOYOBO, SMK-101) with pcDNA3.1-3xFlag-PIWII,1 cDNA as a template, according to the instructions of the manufacturer. PCR primers are listed in Table 2.

### Example 5 PIWIL1 plays its biological function and regulation in gastric cancer in a piRNA-independent manner through the UPF1-mediated nonsense mRNA decay pathway (NMD pathway)

### 5.1 PIWIL1 binds to UPF1-mediated NMD complex

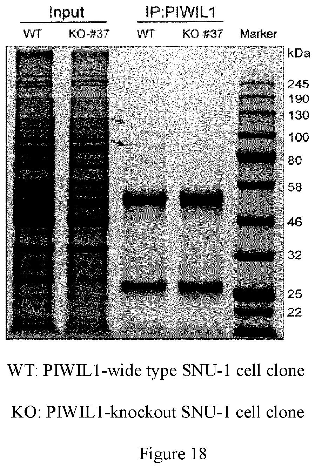

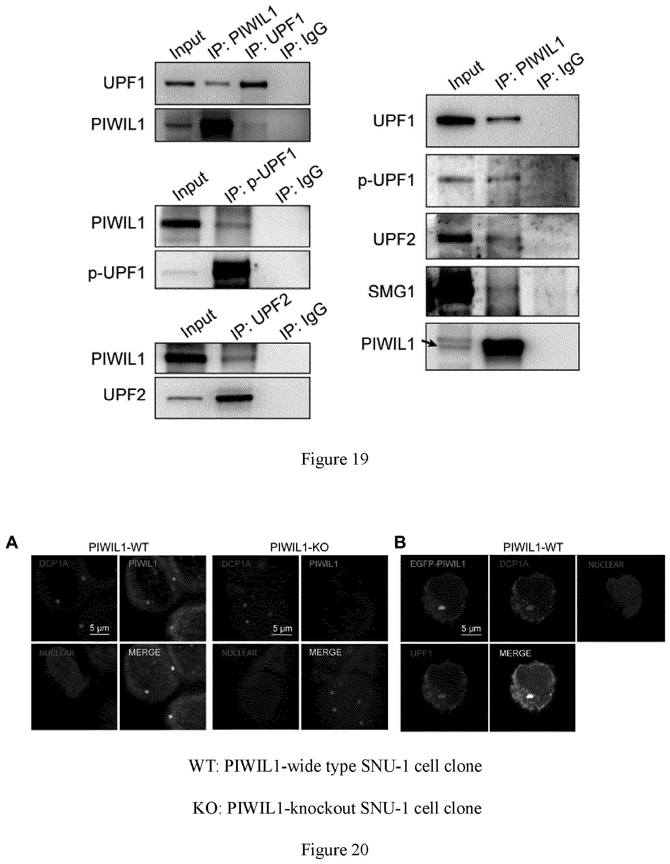

By PIWIL1-immunoprecipitation coupled with mass spectrometry (PIWIL1-IP-MS), we identified UPF1 protein, which is the core protein in the nonsense mRNA decay pathway(36-37)(Figure 18). The results of the immunoprecipitation experiment in Figure 19 further indicate that PIWIL1 and UPF1 bind to each other and that PIWIL1 can bind to the core complex of the NMD pathway (UPF2, SMG1, and activated UPF1 (phosphorylated UPF1)). The results of immunofluorescence assay in Figure 20 shows that PIWIL1 could co-localize with UPF1 in P-bodies, which are organelles of nonsense mRNA decay pathway.

### 5.2 PIWIL1 binds to UPF1 independently of piRNA and has a specific binding region to UPF1

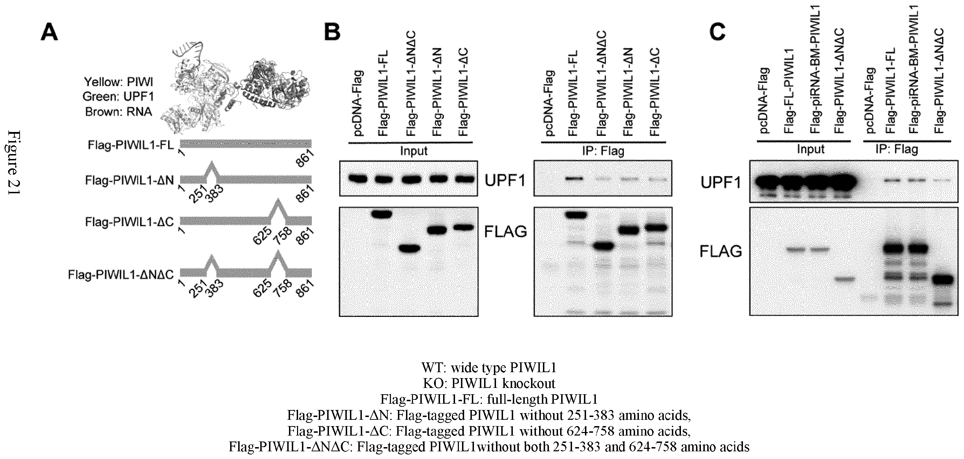

To find out how PIWIL1 specifically binds UPF1 to participate in the NMD pathway, we need to find the binding region where PIWIL1 specifically binds UPF1. The binding pattern ofPIWIL1 to UPF1 was predicted by protein modeling (38-40), based on which we constructed domain mutants of PIWIL1 lacking the ability to bind UPF1. Figure 21 shows that amino acid region 251-383 and amino acid region 625-758 of PIWIL1 are key regions responsible for the binding of PIWIL1 to UPF1. Knockout of this region will significantly disrupt the binding of PIWIL1 to UPF1. However, the PIWIL1 mutant, which only lost piRNA binding ability, was not affected for its binding to UPF1, indicating that the binding of PIWIL1 and UPF1 does not require piRNA for synergistic NMD pathway.

### 5.3 The specific binding of PIWIL1 to UPF1 is essential for the important biological function and regulation of PIWII,1 in gastric cancer

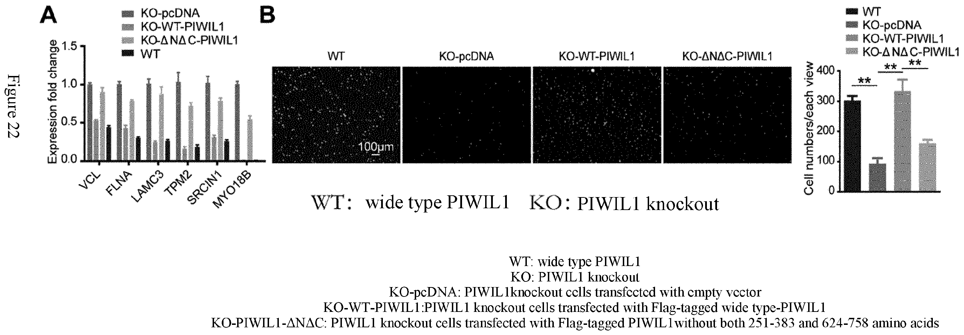

The PIWIL1 mutant lacking the ability to bind UPF1 and the wild-type PIWIL1 were transfected into PIWIL1 knockout gastric cancer cell SNU-1, respectively, it was found that wild-type PIWIL1 could effectively repair the up-regulation of downstream tumor suppressor gene expression and the inhibition of cancer cell migration caused by PIWIL1 knockout, but the loss of the binding ability of PIWIL1 to UPF1 could not restore the up-regulation of downstream tumor suppressor gene expression and the effect on cell migration caused by PIWIL1 knockout(Figure 22).

In conclusion, this Example shows that PIWIL1 can regulate gastric cancer cell migration and tumor suppressor genes related to cell migration through the UPF1-mediated nonsense mRNA decay (NMD pathway ) independently of piRNA. Therefore, the piRNA-independent regulation of PIWII,1, especially the synergistic regulation with the NMD complex, is expected to be a new treatment of PIWIL 1 in the targeted therapy of gastric cancer, which will be different from the traditional piRNA-dependent treatment of PIWIL1.

### Materials and Methods

### Co-immunoprecipitation

Cells were lysed in co-immunoprecipitation lysis buffer [20mM Tris-HCl pH7.4, 150mM NaCl, 1 % (v/v) IGEPAL^{®} CA-630 (Millipore SIGMA, Cat# I8896), 1mM EDTA, 0.5 mM DTT, cOmpleteTM EDTA-free Protease Inhibitor Cocktail Tablets (MERCK, Cat# 4693132001), and PhosStop Tablets (MERCK, 4906837001)], and centrifugedat 14,000 rpm for 10 minutes to remove the debris. 10 µL of empty Dynabeads^{®}Protein G (ThermoFisher Scientific, Cat# 10004D) was added to the lysates and incubated for 0.5 hours for pre-clearing. 50 µL of empty Dynabeads^{®} Protein G was washed by Citrate-Phosphate Buffer (pH 5.0) and then incubated with 5µg mouse monoclonal anti-PIWIL1 antibody (Millipore SIGMA, Cat# SAB4200365) or 5µg normal mouse IgG polyclonal antibody (MERCK, Cat# 12-371). 50 µL of empty Dynabeads^{®} Protein A were washed by Citrate-Phosphate Buffer (pH 5.0) and then incubated with 15µl rabbit monoclonal anti-UPF1 antibody (Cell Signaling TECHNOLOGY, Cat# 12040) or 5µg rabbit polyclonal anti-phosphoUpf1 (Ser1127) antibody (MERCK, Cat# 07-1016) or 5µg rabbit polyclonal anti-UPF2 antibody (Abcam, ab157108) or 5µg normal rabbit IgG polyclonal antibody (MERCK, Cat# 12-370). All these incubations were done at 4°C for 4 hours with rotation. The Dynabeads Protein G/A-Ig complex was washed by IP wash buffer [20mM Tris-HCl pH7.4, 200mM NaCl, 0.05 % (v/v) IGEPAL^{®} CA-630, 0.5 mM DTT, cOmpleteTM EDTA-free Protease Inhibitor Cocktail Tablets, and PhosStop Tablets] three times. The prewashed Dynabeads Protein G/A-Ig complex was incubated with the precleared lysates at 4°C overnight with rotation. The beads were then washed with IP wash buffer twice and IP high-salt wash buffer [20mM Tris-HCl pH7.4, 500mM NaCl, 0.05 % (v/v) IGEPAL^{®} CA-630, 0.5 mM DTT, cOmpleteTM EDTA-free Protease Inhibitor Cocktail Tablets, and PhosStop Tablets] twice. Immunoprecipitates were run on 7.5% TGX Fast Cast acrylamide gels and probed with relevant antibodies for Western blotting or detected using Coomassie Brilliant Blue staining solution based on the manufacturer's instructions.

### Mass spectrometry

The co-immunoprecipitated products were subjected to electrophoresis using 7.5% TGX Fast Cast acrylamide kit (Bio-Rad, 1610173TA) followed by Coomassie Brilliant Blue staining. Protein spots were then cut separately; the excised gel bands were first in-gel digested as described above(41). The obtained peptides were desalted, followed by nano-LC/MS/MS analysis. Orbitrap Fusion MS equipped with nano EASY LC and Easyspray column (75 µm × 50 cm, PepMap RSLC C18 column, 2 µm, 100 Å, ThermoFisher Scientific) was employed to acquire MS data. The LC gradient of was 5 to 35% B (0.1% formic acid in CH₃CN) in 60 min at a flow rate of 300 NL/min, the column temperate was set to 50 °C. The MS data were acquired in datadependent mode. Briefly, a survey scan at 60K resolution is followed by ten HCD MS/MS scans performed in Orbitrap at 15K resolution.

### Molecular construction of a PIWIL1 mutant lacking the ability to bind UPF1

The cloning of PIWIL1-domain mutant was carried out by the Phanta Max Super-Fidelity DNAPolymerase kit (Vazyme, P505-d1) and Mut Express MultiS Fast Mutagenesis Kit V2 (Vazyme, C215-01), according to the instructions of the manufacturer. Primer (2-250)/(2-624)-F' and Primer (2-250)-R' were used to get fragment (2-250); Primer (384-861)/(384-624)-F' and Primer (384-861)/(759-861)-R' were used to get fragment (384-861); Primer (2-250)/(2-624)-F' and Primer (2-624)/(384-624)-R' were used to get fragment (2-624); Primer (759-861)-F' and Primer (384-861)/(759-861)-R' were used to get fragment (759-861); Primer (384-861)/(384-624)-F' and Primer (2-624)/(384-624)-R' were used to get fragment (384-624). The flag-PIWIL1-ΔN mutant cDNA (deletion of 251-383) was constructed by connecting fragment (2-250) and fragment (384-861). The flag-PIWIL1-ΔC mutant cDNA (deletion of 625-758) was constructed by connecting fragments (2-624) and fragment (759-861). The flag-PIWIL1-ΔNΔC mutant cDNA (deletion of 251-383 and 625-758) was constructed by connecting fragment (2-250) to fragment (384-624) and fragment (759-861). PCR primers are listed in Table 2. Quantitative PCR assay and cell migration assay are as described above.

**Table 1 Primer sequences for quantitative PCR**

| **Gene** | **Forward Primer** | **SEQ** | **Reverse Primer** | **SEQ** |
|---|---|---|---|---|
| ACTB | CCCTGGAGAAGAGCTACGAG | 1 | GGAAGGAAGGCTGGAAGAG T | 2 |
| PIWIL1 | CAGCCAAGTCACAAGGACTC | 3 | GATGTCAGCCGGAAATGGTT | 4 |
| PIWIL2 | TTGGTTGGAGTAGGACGCTT | 5 | AAGGTACAGGGAGGCTTGTC | 6 |
| PIWIL4 | GATGGCACCGAGATCACCTA | 7 | TGGTCAGTCAGCCCTGTTAG | 8 |
| UPF1 | ATCCGCCTGCAGGTCCAGTA | 9 | GATCCGCTGCAGTGACACCA | 10 |
| FLNA | GGGATCCCATCCCTAAGAGC | 11 | CTTGGATGCCACTTTGCCTT | 12 |
| LAMC3 | ACTGTGAGCACTGTCAGGAA | 13 | TGCAAGGCATGCATTTGTCT | 14 |
| CCNE2 | GGGGGATCAGTCCTTGCATT | 15 | AAGGCAGCAGCAGTCAGTAT | 16 |
| ORC6 | ATGCTGAGGAAAGCAGAGG A | 17 | TTCATCCAGGAAGCTGCAAG | 18 |
| CDC25A | GTGCCGGTATGTGAGAGAGA | 19 | TGCGGAACTTCTTCAGGTCT | 20 |
| MCM2 | CGAGATAGAGCTGACTGGCA | 21 | CAGTGGCAAAGACAGGGAA G | 22 |
| CCND3 | ATCACTGGCACTGAAGTGGA | 23 | TCTGTAGGAGTGCTGGTCTG | 24 |
| PCNA | GCGTGAACCTCACCAGTATG | 25 | TCTCCTGGTTTGGTGCTTCA | 26 |
| MYO18B | GAAGCAGATGCACCAGAAG G | 27 | CCAATCTGGTCACAGAGGGT | 28 |
| VCL | GACCGGCCAAAGCAGCTGTA | 29 | GATGGCAGCCTGACCGACTC | 30 |
| SESN2 | GGCTGGAGGCACTGATGTCC | 31 | TAGTCAGGGTGCAGGCCCAT | 32 |
| SRCIN1 | ACAGAGCTCAAGGCTCACTT | 33 | GGCTCCTCCTTCAGGAACTT | 34 |
| TPM2 | GATGCTGAAGCTGGACAAGG | 35 | GGTCCTCAGCTTGCTTCTTG | 36 |
| DDIT4 | GAGTCCCTGGACAGCAGCAA | 37 | CAGCAGCTGCATCAGGTTGG | 38 |
| CCDC80 | AGCAGAAGAAGGAGGGCAT T | 39 | AGATCACCAGCAACCTCCTC | 40 |
| PTEN | GCGGAACTTGCAATCCTCAG | 41 | GAACTTGTCTTCCCGTCGTG | 42 |
| GADD45 G | AGCTGCTGGTTGATCGCACT | 43 | AGCAACTCATGCAGCGCTTT | 44 |
| GADD45B | TGAATGTGGACCCAGACAGC | 45 | AAGGACTGGATGAGCGTGAA | 46 |
| TSC2 | TACTGCGTCTGCGACTACAT | 47 | CCAGTCAGACTCCTGCTTCA | 48 |
| TP53 | GTCCAGATGAAGCTCCCAGA | 49 | CAAGAAGCCCAGACGGAAA C | 50 |
| MCM10 | GTGGAAGCCTTCTCTGGTCT | 51 | CAGCTTCTCTCTGGCCATCT | 52 |

**Table 2 PCR primer sequences**

| **Primers ID** | **Sequence(5'to3')** | **SEQ** |
|---|---|---|
| 2U6 hPiwil-1 E5 sg2 For | atgCGTCTCaACCGcattgactataacccactgagttttagagctagaaa tagcaag | 53 |
| 2U6 hPiwil-1 E5 sg1 Rev | atgCGTCTCgAAACgctgacatcccgtccccagtCGGTGTTTC GTCCTTTCCACAAG | 54 |
| hPiwil-1 E5 C9(genomePCR) For | ACTGTGCCTGCCTTGTCAT | 55 |
| hPiwil-1 E5 C9 (genomePCR)Rev | GGTAGTTCTGCTTTGAAGTGGAA | 56 |
| PIWIL1-piRNA-binding mutant For | GCAAAATACCTGTGTACAGATTGCCCTACCCCA AGTGCATGTGTGGTGGCCCGAACCTTAGGCAAA CAGCAAACTGTCATGGCCATTGCTACAAAGATT GCCCTAGCAATGAACT | 57 |
| PIWIL1-piRNA-binding mutant Rev | AATAGCATCGTATTTGTCCTTCCGATTACT | 58 |
| (2-250)/(2-624)-F' | gatgatgacgacaaaggatccACTGGGAGAGCCCGAGCC | 59 |
| (2-250)-R' | tcaggAGGCCAAATCACCAACCTGTG | 60 |
| (384-861)/(384-624)-F' | ttggtgatttggcctCCTGAGCTCTGCTATCTTACAGGTC | 61 |
| (384-861)/(759-861)-R' | aacgggccctctagactcgagTTAGAGGTAGTAAAGGCGGT TTGA | 62 |
| (2-624)/(384-624)-R' | tgttccaggaagtggCTTCAGGGGGATGTCCACCC | 63 |
| (759-861)-F' | tgaagCCACTTCCTGGAACAGTTATTGATG | 64 |

## Claims

1. A medicament that blocks binding of PIWI and NMD complex proteins for one or more of following purposes:
(a)to diagnose and/or treat cancers with high expression of PIWI and/or NMD complex proteins;
(b)to inhibit growth of cancer cells;
(c)to inhibit progression of cancer cell cycle;
(d)to inhibit migration of cancer cells;
(e)to inhibit tumorigenicity of cancer cells;
(f)to inhibit metastasis of cancer cells *in vivo;*
(g)to regulate cell cycle signaling pathways of cancer cells;
(h)to regulate focal adhesion and adherens junction signaling pathways of cancer cells.

2. The medicament of Claim 1,wherein the cancer is gastric cancer, for example, early or advanced gastric cancer;
and/or, the PIWI comprises PIWIL1, PIWIL2, PIWIL3 and/or PIWIL4.
and/or, the NMD complex proteins comprise UPF1, UPF2, SMG1, UPF3, SMG5, SMG6 and/or SMG7, preferably UPF1, UPF2 and/or SMG1.
and/or, the medicament is a medicament that blocks the binding by mutating and/or inhibiting binding site of the PIWI and the NMD complex proteins, preferably by mutating and/or inhibiting the binding site of the PIWI that binds to the NMD complex proteins, and/or preferably a medicament that blocks the binding by mutating and/or inhibiting the binding site of the NMD complex that binds the PIWI.

3. The medicament of Claim 2, wherein the medicament is an inhibitor designed for the binding site of the PIWI and the NMD complex proteins, preferably the inhibitor designed for the binding site of PIWI to the NMD complex proteins, such as PIWIL1 inhibitor, PIWIL2 inhibitor, PIWIL3 inhibitor and/or PIWIL4 inhibitor, and/or, the inhibitors the inhibitor designed for the binding site of NMD complex proteins to the PIWI, such as UPF1 inhibitor, UPF2 inhibitor, SMG1 inhibitor, UPF3 inhibitor, SMG5 inhibitor, SMG6 inhibitor and/or SMG7 inhibitor, preferably UPF1, UPF2 and/or SMG1 inhibitor;
and/or, the medicament is a small molecule substance, for example, a small molecule chemical substance such as a small molecule compound, or a small molecule biological substance such as a small molecule active peptide; the small-molecule compound preferably exists in a form of a pharmacologically acceptable salt of the compound, a solvent complex of the compound, a solvent complex of a pharmacologically acceptable salt of the compound or a crystalline form of the compound;
the medicament is a macromolecular substance, for example, a macromolecular biological substance such as a polysaccharide, a protein such as an antibody, a nucleic acid, or a macromolecular chemical substance such as a polymer compound;
and/or, the medicament is an extract of traditional Chinese medicine and/or an animal or plant.

4. A medicament that targets PIWI and/or NMD complex proteins for one or more of following purposes:
(a)to diagnose and/or treat cancers with high expression of PIWI and/or NMD complex proteins;
(b)to inhibit the growth of cancer cells;
(c)to inhibit the progression of cancer cell cycle;
(d)to inhibit the migration of cancer cells;
(e)to inhibit tumorigenicity of cancer cells;
(f)to inhibit metastasis of cancer cells *in vivo;*
(g)to regulate cell cycle signaling pathways of cancer cells;
(h)to regulate focal adhesion and adherens junction signaling pathways of cancer cells.

5. The medicament of Claim 4, wherein the PIWI comprises PIWIL1, PIWIL2, PIWIL3 and/or PIWIL4;
and/or, the NMD complex proteins are UPF1, UPF2, SMG1, UPF3, SMG5, SMG6 and/or SMG7, preferably UPF1, UPF2 and/or SMG1;
and/or, the medicament that targets PIWI is PIWI inhibitor, such as PIWIL1 inhibitors, PIWIL2 inhibitors, PIWIL3 inhibitors, and/or PIWIL4 inhibitors;
and/or, the medicament that targets NMD complex proteins is inhibitor of NMD complex proteins, such as UPF1 inhibitors, UPF2 inhibitors, SMG1 inhibitors, UPF3 inhibitors, SMG5 inhibitors, SMG6 inhibitors and/or SMG7 inhibitors, such as NMDI-14, NMDI1 and/or VG1;
and/or, the cancer is gastric cancer, such as early or advanced gastric cancer;
and/or, the medicament is a small molecule substance, for example, a small molecule chemical substance such as a small molecule compound, or a small molecule biological substance such as a small molecule active peptide;
and/or, the medicament is a macromolecular substance, for example, a macromolecular biological substance such as a polysaccharide, a protein such as an antibody, a nucleic acid, or a macromolecular chemical substance such as a polymer compound;
and/or, the medicament is an extract of traditional Chinese medicine and/or an animal or plant.

6. A medicament that reduces expression of PIWI and/or NMD complex proteins for one or more of following uses:
(a) to diagnose and/or treat cancers with high expression of PIWI and/or NMD complex proteins;
(b)to inhibit growth of cancer cells;
(c)to inhibit progression of cancer cell cycle;
(d)to inhibit migration of cancer cells;
(e)to inhibit tumorigenicity of cancer cells;
(f)to inhibit metastasis of cancer cells *in vivo;*
(g)to regulate cell cycle signaling pathways of cancer cells;
(h)to regulate focal adhesion and adherens junction signaling pathways of cancer cells.

7. The medicament of Claim 6, wherein the medicament that reduces PIWI expression is a medicament that silences, downregulates, and/or knocks out PIWI gene;
and/or, the medicament that reduces the expression of NMD complex proteins is medicament that silences, downregulates, and/or knocks out genes of NMD complex proteins;
and/or, the PIWI comprises PIWIL1, PIWIL2, PIWIL3 and/or PIWIL4;
and/or, the NMD complex proteins comprise UPF1, UPF2, SMG1, UPF3, SMG5, SMG6 and/or SMG7, preferably UPF1, UPF2 and/or SMG1;
and/or, the cancer is gastric cancer, such as early or advanced gastric cancer;
and/or, the medicament is a small molecule substance, for example, a small molecule chemical substance such as a small molecule compound, or a small molecule biological substance such as a small molecule active peptide; the small-molecule compound preferably exists in a form of a pharmacologically acceptable salt of the compound, a solvent complex of the compound, a solvent complex of a pharmacologically acceptable salt of the compound, or a crystalline form of the compound;
and/or, the medicament is a macromolecular substance for example, a macromolecular biological substance such as a polysaccharide, a protein such as an antibody, a nucleic acid, or a macromolecular chemical substance such as a polymer compound;
and/or, the medicament is an extract of traditional Chinese medicine and/or an animal or plant.

8. A pharmaceutical composition comprising the medicament of any one of Claims 1-7.

9. The pharmaceutical composition of Claim 8, wherein the pharmaceutical composition is used for one or more of the following purposes:
(a)to diagnose and/or treat cancers with high expression of PIWI and/or NMD complex proteins;
(b)to inhibit growth of cancer cells;
(c)to inhibit progression of cancer cell cycle;
(d)to inhibit migration of cancer cells;
(e)to inhibit tumorigenicity of cancer cells;
(f)to inhibit metastasis of cancer cells *in vivo;*
(g)to regulate cell cycle pathways of cancer cells;
(h)to regulate focal adhesion and adherens junction signaling pathways of cancer cells.
preferably:
the PIWI comprises PIWIL1, PIWIL2, PIWIL3 and/or PIWIL4; and/or, the NMD complex proteins comprise UPF1, UPF2, SMG1, UPF3, SMG5, SMG6 and/or SMG7, preferably UPF1, UPF2 and/or SMG1; and/or, the cancer is gastric cancer, such as early or advanced gastric cancer.

10. The pharmaceutical composition of claim 8 or 9, wherein the pharmaceutical composition does not comprise medicament that targets piRNA, such as piRNA inhibitors; and/or, the pharmaceutical composition does not comprise medicament that blocks binding of PIWI and piRNA;
preferably:
the medicament that blocks binding of PIWI and piRNA is a medicament that blocks binding of PIWI and piRNA by mutating and/or inhibiting binding site of PIWI and piRNA, preferably a medicament that blocks the binding by mutating and/or inhibiting the binding site of the PIWI that binds to the piRNA and/or a medicament that blocks the binding by mutating and/or inhibiting the binding site of the piRNA that binds to the PIWI;
more preferably:
the medicament that blocks the binding of PIWI and piRNA is an inhibitor designed for the binding site of PIWI and piRNA, preferably an inhibitor designed for the binding site of PIWI that binds to the piRNA, and/or an inhibitor designed for the binding site of piRNA that binds to the PIWI.

11. The pharmaceutical composition of any one of Claims 8-10, wherein the pharmaceutical composition further comprises pharmaceutic adjuvant;
and/or, the pharmaceutical composition further comprises other medicaments; the other medicaments are medicaments for the diagnosis and/or treatment of cancers with high expression of PIWI and/or NMD complex proteins;
and/or, the pharmaceutical composition consists of one or more of the medicaments of any one of Claims 1-7.

12. A method for diagnosing and/or treating cancers with high expression of PIWI and/or NMD complex proteins, wherein the method comprises diagnosis and/or treatment using the medicament of any one of Claims 1-7 and/or the pharmaceutical composition of any one of Claims 8-11;
preferably:
the cancer is gastric cancer, such as early or advanced gastric cancer;
and/or, the PIWI comprises PIWIL1, PIWIL2, PIWIL3 and/or PIWIL4.

13. The method of Claim 12, wherein the medicament and/or pharmaceutical composition is used for diagnosis and/or treatment by one or more of following mechanisms:
(a)to inhibit growth of cancer cells;
(b)to inhibit progression of cancer cell cycle;
(c)to inhibit migration of cancer cells;
(d)to inhibit tumorigenicity of cancer cells;
(e)to inhibit metastasis of cancer cells *in vivo;*
(f)to regulate cell cycle pathways of cancer cells;
(g)to regulate focal adhesion and adherens junction signaling pathways of cancer cells.

14. A use of the medicament of Claims 1-7 and/or the pharmaceutical composition of Claims 8-11 in diagnosis and/or treatment of cancers with high expression of PIWI and/or NMD complex proteins;
preferably:
the cancer is gastric cancer, for example, early or advanced gastric cancer;
and/or, the PIWI comprises PIWIL1, PIWIL2, PIWIL3 and/or PIWIL4;
and/or, the medicament and/or pharmaceutical composition is used for diagnosis and/or treatment by one or more of following mechanisms:
(a)to inhibit growth of cancer cells;
(b)to inhibit progression of cancer cell cycle;
(c)to inhibit migration of cancer cells;
(d)to inhibit tumorigenicity of cancer cells;
(e)to inhibit metastasis of cancer cells *in vivo;*
(f)to regulate cell cycle signaling pathways of cancer cells;
(g)to regulate focal adhesion and adherens junction signaling pathways of cancer cells.

15. A use of the medicament of Claims 1-7 and/or the pharmaceutical composition of Claims 8-11 in preparation of a drug for diagnosis and/or treatment of cancers with high expression of PIWI and/or NMD complex proteins;
preferably:
the cancer is gastric cancer, for example, early or advanced gastric cancer;
and/or, the PIWI comprises PIWIL1, PIWIL2, PIWIL3 and/or PIWIL4;
and/or, the medicament and/or pharmaceutical composition is used for diagnosis and/or treatment by one or more of following mechanisms:
(a)to inhibit growth of cancer cells;
(b)to inhibit progression of cancer cell cycle;
(c)to inhibit migration of cancer cells;
(d)to inhibit tumorigenicity of cancer cells;
(e)to inhibit metastasis of cancer cells *in vivo;*
(f)to regulate cell cycle signaling pathways and focal adhesion and adherens junction signaling pathways of cancer cells;

16. A method for inhibiting growth of cancer cells, inhibiting operation of cancer cell cycle, inhibiting migration of cancer cells, inhibiting tumorigenesis ability of cancer, inhibiting metastasis ability of cancer, regulating signaling pathways of cancer cell cycle and/or focal adhesion and adherens junction signaling pathways of cancer cells, wherein the method comprises inhibition and/or regulation by the medicament of any one of Claims 1-7 and/or the pharmaceutical composition of any one of Claims 8-11;
preferably, the cancer is gastric cancer, such as early or advanced gastric cancer; and/or, the PIWI comprises PIWIL1, PIWIL2, PIWIL3 and/or PIWIL4.

17. A use of the medicament of any one of Claims 1-7 and/or the pharmaceutical composition of any one of Claims 8-11 in inhibiting growth of cancer cells, inhibiting operation of cancer cell cycle, inhibiting migration of cancer cells, inhibiting tumorigenesis ability of cancer, inhibiting metastasis ability of cancer, regulating signaling pathways of cancer cell cycle and/or focal adhesion and adherens junction signaling pathways of cancer cells;
preferably, the cancer is gastric cancer, such as early or advanced gastric cancer;
and/or, the PIWI comprises PIWIL1, PIWIL2, PIWIL3 and/or PIWIL4.

18. A use of the medicament of any one of Claims 1-7 and/or the pharmaceutical composition of any one of Claims 8-11 in preparing a drug for inhibiting growth of cancer cells, inhibiting operation of cancer cell cycle, inhibiting migration of cancer cells, inhibiting tumorigenesis ability of cancer, inhibiting metastasis ability of cancer, regulating signaling pathways of cancer cell cycle and/or focal adhesion and adherens junction signaling pathways of cancer cells;
preferably, the cancer is gastric cancer, such as early or advanced gastric cancer;
and/or, the PIWI comprises PIWIL1, PIWIL2, PIWIL3 and/or PIWIL4.

19. A use of PIWI and/or NMD complex proteins in screening of the medicament of any one of Claims 1-7 and/or the pharmaceutical composition of any one of Claims 8-11;
preferably:
the PIWI comprises PIWIL1, PIWIL2, PIWIL3 and/or PIWIL4;
and/or, the NMD complex proteins comprise UPF1, UPF2, SMG1, UPF3, SMG5, SMG6 and/or SMG7, preferably UPF1, UPF2 and/or SMG1.

20. A use of cell lines with high expression of PIWI and/or NMD complex proteins for screening the medicament of any one of Claims 1-7 and/or the pharmaceutical composition of any one of Claims 8-11;
preferably:
the medicament is a small molecule substance, for example, a small molecule chemical substance such as a small molecule compound, or a small molecule biological substance such as a small molecule active peptide; the small-molecule compound preferably exists in a form of a pharmacologically acceptable salt of the compound, a solvent complex of the compound, a solvent complex of a pharmacologically acceptable salt of the compound, or a crystalline form of the compound;
and/or, the medicament is a macromolecular substance, for example, a macromolecular biological substance such as a polysaccharide, a protein such as an antibody, a nucleic acid, or a macromolecular chemical substance such as a polymer compound;
and/or, the medicament is an extract of traditional Chinese medicine and/or an animal or plant.
and/or, the cell lines are gastric cancer cell lines, preferably SNU-1, SNU-16 and/or AGS;
and/or, the PIWI comprises PIWIL1, PIWIL2, PIWIL3 and/or PIWIL4;
and/or, the NMD complex proteins comprise UPF1, UPF2, SMG1, UPF3, SMG5, SMG6 and/or SMG7, preferably UPF1, UPF2 and/or SMG1.

21. A use of PIWI and/or NMD complex proteins as biomarkers and/or therapeutic targets for cancers with high expression of PIWI and/or NMD complex proteins;
preferably, the PIWI comprises PIWIL1, PIWIL2, PIWIL3 and/or PIWIL4; and/or, the NMD complex proteins are UPF1, UPF2, SMG1, UPF3, SMG5, SMG6 and/or SMG7, preferably UPF1, UPF2 and/or SMG1, and/or, the cancer is gastric cancer, such as early or advanced gastric cancer.

22. A biomarker for detection and/or diagnosis of cancers with high expression of PIWI and/or NMD complex proteins, wherein the biomarker comprises PIWI and/or NMD complex proteins;
preferably, the PIWI comprises PIWIL1, PIWIL2, PIWIL3 and/or PIWIL4; and/or, the NMD complex proteins are UPF1, UPF2, SMG1, UPF3, SMG5, SMG6 and/or SMG7, preferably UPF1, UPF2 and/or SMG1, and/or, the cancer is gastric cancer, such as early or advanced gastric cancer.

23. A kit for detection and/or diagnosis of cancers with high expression of PIWI and/or NMD complex proteins, wherein the kit comprises antibodies for detection of PIWI and/or NMD complex proteins;
preferably, the PIWI comprises PIWIL1, PIWIL2, PIWIL3 and/or PIWIL4; and/or, the NMD complex proteins are UPF1, UPF2, SMG1, UPF3, SMG5, SMG6 and/or SMG7, preferably UPF1, UPF2 and/or SMG1, and/or, the cancer is gastric cancer, such as early or advanced gastric cancer.
